(19)

Europäisches
Patentamt
European
Patent Office
Office européen
des brevets

(11)  **EP 2 924 600 A1**

(12)  **EUROPEAN PATENT APPLICATION**

(43) Date of publication:
**30.09.2015 Bulletin 2015/40**

(51) Int Cl.:
***G06F 19/00*** *(2011.01)*     ***G06F 19/12*** *(2011.01)*

(21) Application number: **14161717.5**

(22) Date of filing: **26.03.2014**

| | |
|---|---|
| (84) Designated Contracting States:<br>**AL AT BE BG CH CY CZ DE DK EE ES FI FR GB<br>GR HR HU IE IS IT LI LT LU LV MC MK MT NL NO<br>PL PT RO RS SE SI SK SM TR**<br>Designated Extension States:<br>**BA ME**<br><br>(71) Applicant: **Noori, Hamid Reza<br>68159 Mannheim (DE)** | (72) Inventor: **Noori, Hamid Reza<br>68159 Mannheim (DE)**<br><br>(74) Representative: **Jacobi, Markus Alexander<br>Isenbruck Bösl Hörschler LLP<br>Patentanwälte<br>Eastsite One<br>Seckenheimer Landstraße 4<br>68163 Mannheim (DE)** |

(54)  **A method for determining neurocircuitries and effects of neuroactive compounds**

(57)  The present invention relates to a computer-implemented method for providing a set of clinical data on extracellular local concentrations of one or more neurotransmitter(s) in the absence or presence of one or more neuroactive compound(s) in a set of neuronal regions. Furthermore, the present invention relates to a computer program product for implementing the determination of the neurotransmitter concentration according to said method. Moreover, the present invention further relates to a neuroactive compound for use in a method for treating or preventing a subject suffering from or being at risk of a neuro- and psychopathologic condition.

EP 2 924 600 A1

**Description**

The present invention relates to a computer-implemented method for providing a set of clinical data on extracellular local concentrations of one or more neurotransmitter(s) in the absence or presence of one or more neuroactive compound(s) in a set of neuronal regions. Furthermore, the present invention relates to a computer program product for implementing the determination of the neurotransmitter concentration according to said method. Moreover, the present invention further relates to a neuroactive compound for use in a method for treating or preventing a subject suffering from or being at risk of a neuro- and psychopathologic condition.

Alterations of extracellular local concentrations of neurotransmitters in the nervous system are widely acknowledged as robust *in vivo* indicators of a subject's health status and of acute and chronic neuroactive compound effects. In particular, such extracellular local concentrations of neurotransmitters are considered to provide appropriate measures for the efficacy of neuropsychiatric neuroactive compounds.

Therefore, the development of technologies to determine the dynamics of neuroactive compound-induced neurochemical changes has been one of the main focusses of both scientific research and pharmaceutical industry. Today a variety of invasive and/or non-invasive methods based on, e.g., microdialysis (in particular dual-probe), voltammetry, proteomics, magnetic resonance tomography (MRT), pharmacological MRT based on local cerebral blood perfusion, positron emission tomography (PET), single-photon emission computed tomography (SPECT) and/or other biosensors, is commonly employed for this purpose. All of these methods, however, share significant drawbacks.

Firstly, those invasive methods are typically restricted to single- or dual-region observations in a tissue. Investigators are, therefore, often forced to content themselves with a few snapshots of neurotransmitter alterations in at most two neuronal regions in order to estimate the systematic effects of neuroactive compounds. This limitation severely impedes the untangling of more complex patterns of neurochemical connectivities (neurocircuitries).

Secondly, untangling more complex patterns of neurocircuitries, if possible at all, by the methods known in the art, typically requires a stepwise proceeding meaning that a large number of animals needs to be sacrificed and thereby typically leads to high efforts in both financial and labor expenses and may provoke ethical constraints. Typically, for each extracellular concentration of each neurotransmitter in each neuronal region of interest, at least eight to ten animals are required. The investigation of dose-dependency of a neuroactive compound (neuroactive compound-dose scenario) or even combinations of neuroactive compounds (neuroactive compound-neuroactive compound scenario) requires even higher efforts. Moreover, side-effects are often overseen as the measurement is merely focused on few neuronal interconnections, therefore, an extremely simplified neurocircuitry model.

Finally, those invasive methods typically require physical access to the tissue of interest inevitably associated with at least partly tissue damage, which in turn sets natural limits for the number of regions in a subject that can be observed simultaneously as well as for the duration and time-scale of measurements. Further, notably, not all regions in a subject such as, e.g., brain nuclei, are accessible without harming the subject severely. Further, those invasive methods typically require long-lasting and laborious experimentation.

In summary, the methods for determining neurocircuitries known in the art so far bear significant drawbacks both from an economical and from an ethical point of view, the applicability of said methods in restricted and the informative value of the experimental results is limited. Therefore, it had been tried to use assumption-free construction models to simulate neurocircuitries (Noori H.R. et al. (2012), Addict Biol 17(5):827-864; Noori H.R., (2011), Math Med Biol 29(3):231-244; Noori H.R. and Jäger W., (2010), Bull Math Biol 72(1):133-147).

These methods, however, still bear significant drawbacks as they do not enable to determine the exact pharmacologically and toxicologically effects of neuroactive compounds interacting with the neurocircuitries and do not reflect the neuronal networks in detail, in particular do not enable effective rescaling of the determined values to measured values. Therefore, the informative value of these methods is still limited.

In the view of the above, there is still an unmet need for methods for overcoming one or more of the above drawbacks. Surprisingly, it has been found that by means of the computer-implemented method of the present invention as laid out below, a set of only few clinical data can be expanded, thereby enabling the determination of considerably complex neurocircuitries in the presence or absence of neuroactive compound(s), optionally in a time-resolved manner. This can also be provided for a human-like system. Moreover, application of the method of the present invention may also significantly reduce the number of animals required and, thereby, limits costs, labor efforts and ethical constraints.

In a first aspect, the present invention relates to a computer-implemented method for providing a set of clinical data on extracellular local concentrations of one or more neurotransmitter(s) in the absence or presence of one or more neuroactive compound(s) in a set of neuronal regions, said method comprising the steps of:

(i) providing clinical data on evolution of extracellular local concentrations of the one or more neurotransmitter(s) in the absence and/or presence of neuroactive compounds in a reduced number of neuronal regions from the set of neuronal regions;
(ii) optionally, providing the initial concentration(s) of the one or more neuroactive compound(s) in each neuronal region of the set of neuronal regions;

(iii) simulating an evolution of extracellular local concentrations of one or more neurotransmitters within a neurocircuitry modelling the extracellular local concentrations of said one or more neurotransmitters in the absence or optionally in the presence of neuroactive compounds and the effect of said initial concentrations of said one or more neuroactive compounds,

wherein the basal extracellular local concentration(s) of said one or more neurotransmitter(s) are averaged basal extracellular local concentration(s); and

(iv) rescaling the simulated evolution of extracellular local concentrations of the one or more neurotransmitter(s) by the clinical data on evolution of extracellular local concentrations of the one or more neurotransmitter(s) in the absence and/or presence of neuroactive compounds in a reduced number of neuronal regions,

whereby the clinical data on evolution of extracellular local concentrations of the one or more neurotransmitter(s) in the absence and/or presence of neuroactive compounds in the reduced number of neuronal regions is extended to clinical data on extracellular local concentrations of the one or more neurotransmitter(s) in the absence or presence of one or more neuroactive compound(s) for each neuronal region of the set of neuronal regions.

Additionally or alternatively, the rescaling the simulated evolution of extracellular local concentrations of the one or more neurotransmitters by the clinical data on evolution of blood oxygen and/or glucose levels in the absence and/or presence of neuroactive compounds in a reduced number of neuronal regions, whereby the clinical data on evolution of blood oxygen and/or glucose levels in the absence and/or presence of neuroactive compounds in the reduced number of neuronal regions is extended to clinical data on extracellular local concentrations of the one or more neurotransmitters in the absence or presence of one or more neuroactive compounds for each neuronal region of the set of neuronal regions.

This method may provide 4D trajectories (such as 3D location of a neuronal region including the neurotransmitter concentration x time) of the dynamics of neurotransmitter concentrations for multiple neurotransmitter systems in each neuronal region with no temporal limitations. Long-term observations of neurochemical responses to effects to the administration to neuroactive compounds, even for several years, are optionally manageable with a high resolution such as, e.g., a resolution of nM x ms.

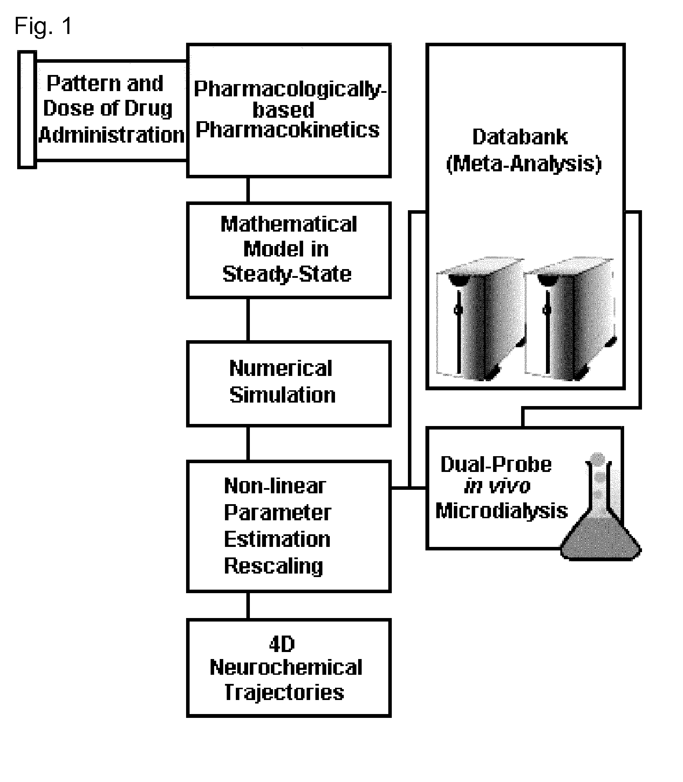

The method according to the present invention may utilize consecutive interacting computational and experimental steps as exemplified and summarized in Figure 1. Herein, simplified, the method may comprise the following steps:

(i) providing an extracellular local concentration for a condition of interest (e.g., dependent on the health status of the investigated subject and optional neuroactive compound administration (combination of neuroactive compound(s), dose, administration scheme and route of administration thereof);

(ii) integrating the extracellular local concentration within the framework of a mathematical model, which may be comprised by a system of delay-differential equations;

(iii) providing numerical simulations of the mathematical model reflecting the qualitative changes of the extracellular local neurotransmitter concentrations;

(iv) rescaling the simulated numerical trajectories of the neuroactive compound-induced dynamical behavior of neurotransmitter concentrations by non-linear parameter estimations into quantitative values in a similar manner as the *in vivo* experiments (e.g., by means of microdialysis (in particular dual-probe) and voltammetry, proteomics, magnetic resonance tomography (MRT), pharmacological MRT based on local cerebral blood perfusion, positron emission tomography (PET), single-photon emission computed tomography (SPECT) and/or other biosensors).

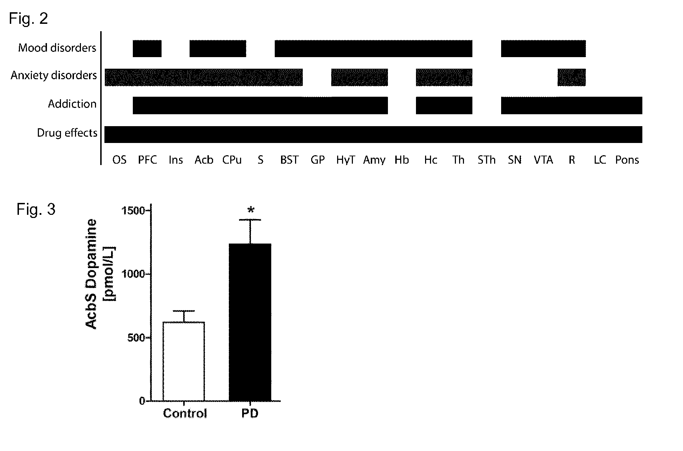

(v) providing the rescaled dynamical trajectories of the extracellular local concentrations of the neuroactive compound-induced neurotransmitter for all neuronal regions within the neurocircuitry for modeling neuroactive compound effects as optionally depicted in Figure 2.

The person skilled in the art will notice that the method according to the present invention may also be used for the screening of compounds, in order to provide new and/or improved neuroactive compounds, combinations of neuroactive compounds, applications, pharmaceutical dosage regimes and/or administration routes. Further, also new therapeutic applications may be determined for known compounds so far used for other therapeutic indications, i.e., in the context of compound repurposing.

In general, the method is conductible on any kind of computer. Computer as used herein may be understood in the broadest sense as any device that is programmable to carry out a set of arithmetic and/or logical operations. Typically, a computer comprises at least one processing element and some form of memory.

As used throughout the present invention, the term "set" may be understood in the broadest sense as any compilation or assembly of at least two, preferably three, in particular more than three of a kind.

As used herein, the term "clinical data" may be understood in the broadest sense as values that were or could be determined, assessed or expected to be present in a subject. Exemplarily, a set of clinical data on extracellular local concentrations of one or more neurotransmitter(s) refers to the extracellular local concentrations of one or more neurotransmitter(s) that could be determined, assessed or expected to be present in the extracellular space of a subject. Additionally or alternatively, a set of clinical data may represent the local blood oxygen level and/or blood glucose concentration in one or more neuronal region(s), in particular brain region(s).

Optionally, the clinical data rely on experimentally obtained values (e.g., determined by *in vivo* microdialysis (in particular dual-probe), voltammetry, proteomics, magnetic resonance tomography (MRT), pharmacological MRT based on local cerebral blood perfusion, positron emission tomography (PET), single-photon emission computed tomography (SPECT) and/or by means of other biosensors) in at least one neuronal region and three doses, two neuronal regions and two doses or alternatively one dose of the neuroactive compound with acute and chronic measurements of the time-course neuroactive compound-induced alterations of the extracellular local concentrations of the neurotransmitter.

The methods of *in vivo* microdialysis (in particular dual-probe) and voltammetry may be considered as (partly) invasive methods as these typically include insertion of one or more probe(s) into the neuronal region(s) of interest. In contrast, magnetic resonance tomography (MRT), pharmacological MRT based on local cerebral blood perfusion, positron emission tomography (PET), single-photon emission computed tomography (SPECT) and/or the use of other biosensors) may be considered as non-invasive methods as herein, typically no device or probe is inserted into body. MRT may include the administration of isotope- and/or metal-labeled neurotransmitter(s) and/or neuroactive compound(s) to the subject. PET may include the administration of radioactively (positron-emitting) neurotransmitter(s) and/or neuroactive compound(s) to the subject.

A biosensor may exemplarily be an antibody or antibody fragment selectively binding to the neurotransmitter or an hapten formed by the neurotransmitter and a high-molecular weight structure (e.g., a neurotransmitter- neurotransmitter receptor complex), wherein said antibody or antibody fragment may be labelled (e.g., fluorescently-, isotope-, metal- and/or radioactively labelled) and/or may be bound by a second antibody or antibody fragment that is labelled. The person skilled in the art will know how to apply such methods in detail and how to experimentally determine the local extracellular concentration of a neurotransmitter.

The experimental measuring step may or may not form a step of the present method. This experimental step may, however, be omitted if the required trajectories are available in a databank (e.g. stored as 4D-trajectories for neuroactive compounds) of previously performed microdialysis experiments. If the method is intended to estimate a novel neuroactive compound, then, exemplarily, a dual-probe microdialysis experiment may be performed on the subject of interest (e.g., a rat), which in turn may be further included in the databank for future references.

Therefore, the method may optionally assisted by *in vivo* measurements (e.g., by means of microdialysis (in particular dual-probe), voltammetry, magnetic resonance tomography (MRT), pharmacological MRT based on local cerebral blood perfusion, positron emission tomography (PET), single-photon emission computed tomography (SPECT) and/or other biosensors) and/or neuroimaging performed on a spatial scale to differentiate between different neuronal regions.

This may comprise the complete network structure of all or some previously introduced neurocircuitries for mood disorders, anxiety disorders and addiction (Figure 2) and may, thereby, provide an appropriate framework to investigate co-morbidities, interactions of different classes of neuroactive compounds and ultimately neuroactive compound-repurposing.

The terms "subject" and "individual" as used herein may be understood interchangeably in the broadest sense as any living being comprising at least one neuronal region in which the extracellular local concentration may be determined according to the present invention. The subject may also be understood as "patient", wherein said subject is not necessarily at a diseased state. The subject may preferably be an animal. More preferably, the subject is a mammal, even more preferably a primate or a rodent, in particular a human, a rat or a mouse. The subject may also be any other animal of interest such as, e.g., a bovine, a horse, a sheep, a goat, a rabbit, a dog, a cat and also a fish, an insect, a crustacean, an arachnidan etc.

As used herein, the term "neurotransmitter" (in the formulas of the present invention indicated as k) may be understood in the broadest sense as any endogenous molecule that enables transmission of signals from a neuron to a target cell across a synapse (synaptic transmission). Herein, the neurotransmitter may be any neurotransmitter known in the art.

Often, a neurotransmitter as used herein is selected from the group consisting of:

    (a) an amino acid selected from the group consisting of glutamate (Glu), gamma-aminobutyrate (GABA), aspartate (Asp) and glycine (Gly);

    (b) a monoamine selected from the group consisting of norepinephrine (NE, noradrenaline), dopamine (DA), serotonin (5-HT, 5-hydroxytryptamine), epinephrine (adrenaline), octopamine, tyramine and melatonin;

    (c) acetylcholine (ACh);

    (d) a diamine, in particular histamine;

(e) an opioid selected from the group consisting of corticotrophin, dynorphin, endorphin and enkephaline;

(f) a secretin selected from the group consisting of secretin, motilin, glucagon, vasoactive intestinal peptide and growth hormone-releasing factor;

(g) a neuropeptide selected from the group consisting of N-acetylaspartylglutamate, neuropeptide Y, pancreatic polypeptide, peptide YY, vasopressin (ADH), oxytocin, tachykinine, cholecystokinine, neurotensin, neurophysin I and neurophysin II;

(h) one or more gaseous neurotransmitters(s) selected from the group consisting of nitric oxide (NO) and carbon monoxide (CO);

(i) a purine phosphoric ester, preferably a purine phosphoric ester selected from the group consisting of adenosine triphosphate (ATP), adenosine diphosphate (ADP), adenosine monophosphate (AMP), uracil triphosphate (UTP) and uracil diphosphate (UDP); and

(j) a neurotransmitter selected from the group consisting of gastrin, cholecystokinin, somatostatin (SIH), neurokinin A, neurokinin B, substance P, bombesin, gastrin releasing peptide, and anandamide.

Preferably, a neurotransmitter as used herein is selected from the group consisting of an amino acid (e.g., glutamate (Glu), gamma-aminobutyrate (GABA), aspartate (Asp), glycine (Gly)), a monoamine (e.g., norepinephrine (NE), dopamine (DA), serotonin (5-HT), epinephrine (adrenaline), octopamine, tyramine, melatonin) and acetylcholine (ACh).

Particularly preferably, a neurotransmitter as used herein is selected from the group consisting of glutamate (Glu), gamma-aminobutyrate (GABA), acetylcholine (ACh), dopamine (DA), norepinephrine (NE) and serotonin (5-HT).

In a whole animal or human body, many different types of neurotransmitters typically interact with another in an interconnected neural network. Therefore there are a number of neurochemical trajectories. This physiological process may also be understood as neurochemical connectivity. The patterns of neurochemical connectivities, optionally influencing themselves, may be understood as neurocircuitries (i.e., systems of neurocircuits, system of neural circuits). Neurochemical connectivity may be understood in the broadest sense as any interaction of an extracellular local concentration of a neurotransmitter k with the extracellular local concentration of the same or other type of neurotransmitter k in another region and/or the extracellular local concentration of another type of neurotransmitter in the same or another region.

A neurotransmitter may be generated and/or stored in a cell and released into an extracellular space upon stimulation of said cell. Such extracellular space may be any space outside the cells. Exemplarily, the extracellular space may be the space outside the cells in a whole region of the peripheral or central nervous system (CNS). Preferably, the space outside the cells is the located in the CNS. Highly preferably, the neuronal region is located in the brain, i.e. is a brain region, even more preferably a mammal brain region, in particular a human or rodent brain region.

Alternatively, the extracellular space may be a single synaptic cleft in the peripheral or central nervous system or at a motoneuron. Then, the cell that is able to release the neurotransmitter may be a terminal axon.

Independent on whether the extracellular space is a whole region of the peripheral or central nervous system or a single synaptic cleft, the neurotransmitter once released into the extracellular space may bind to one or more type(s) of receptors present on the same or another cell (e.g., a dendrite or a muscle cell) and evoke a cellular response (e.g., increase or decrease of the membrane potential, influx and/or efflux of ion(s), release of other or the same neurotransmitter(s) and/or muscle contraction). For decaying the cellular response, typically, the extracellular local concentration of the neurotransmitter will be decreased.

To decrease the extracellular local concentration of the neurotransmitter, the neurotransmitter may be, exemplarily, resorbed by the cell it was precedingly released from and/or by another cell, may be degraded and/or may diffuse into neighboring regions and thereby lower by means of dilution. On the other hand, concomitantly, the extracellular local concentration of the neurotransmitter may be further increased by further release of said neurotransmitter into said extracellular space and/or by entering into said extracellular space by means of diffusion from neighboring from one or more neighboring region(s) such as from adjacent or proximal region(s) such as, e.g., adjacent or proximal synapse(s). As used herein, neighboring region(s), if any, are indicated by *j*.

When the extracellular local concentration of the neurotransmitter is decreased, the cellular response decays. To enable subsequent further cellular responses, the neurotransmitter concentration will, typically, be restored such as, e.g., by means of synthetical regeneration of the neurotransmitter. Finally, a balance between such physiological processes increasing the extracellular local concentration of the neurotransmitter and those decreasing the extracellular local concentration of the neurotransmitter lead to the formation of a basal extracellular local concentration.

As used herein, the term "extracellular local concentration" may be understood in the broadest sense as the concentration of a neurotransmitter present in an extracellular space, i.e., outside of cells, in a particular neuronal region.

As used herein, in the context of specifying a region of the animal or human body, of an organ or tissue thereof or of a cell, the terms "neuronal region", "neuronal location" and "area in the nervous system" and similar terms may be understood in the broadest sense as any spatial part of the nervous system of an animal or a human body associated with a neuronal network, wherein the extracellular local concentration of a neurotransmitter can be determined.

Preferably, the neuronal region and is a region in the central nervous system (CNS) including the brain, in particular in a brain region. Exemplarily, a brain region may be understood as a spatial part located in a particular position in the brain. Preferably, on the level of an organ (e.g., a brain), a region designates a functional entity of said organ. Exemplarily, the organ of interest is a brain, preferably a mammal brain, in particular a human or rodent (e.g., rat or mouse) brain. A neuronal region in the brain (brain region) may preferably be the olfactory bulb (OB), the prefrontal cortex (PFC), the insula (Ins), the nucleus accumbens (Acb), the caudate putamen (CPu), the septal region (S), the bed nucleus of stria terminalis (BST), the globus pallidus (GP), the hypothalamus (HyT), the amygdala (Amy), the habenula (Hb), the hippocampus (Hc), the thalamus (Th), the subthalamic nucleus (STh), the substantia nigra (SN), the ventral tegmental area (VTA), the raphe nucleus (R), the locus coerulus (LC) and/or pontine nuclei (Pons). Each region may be interconnected with one or more other region(s) via neurochemical trajectories.

Alternatively or additionally, the neuronal region may also be a muscle, in particular one or more neuromuscular junction(s) and/or one or more synapse(s) of the peripheral nervous system.

A reduced number of neuronal regions may be understood as a lower number compared to the number of neuronal regions comprised in the set of neuronal regions. When the set of neuronal regions, exemplarily, comprises ten neuronal regions, the reduced number of neuronal regions may comprise one, two, three, four, five, six, seven, eight or nine neuronal regions. Preferably, the reduced number of neuronal regions may comprise at least two neuronal regions. More preferably, the reduced number of neuronal regions may comprise between two neuronal regions and half of the number of neuronal regions comprised in the set of neuronal regions, therefore, in the aforementioned example, between two and five neuronal regions.

According to the method according to the present invention, the reduced number of neuronal regions preferably comprises at least two neuronal regions, wherein the extracellular local concentrations of at least three neurotransmitters are determined, and wherein punctual measurements are typically sufficient. Preferably, however, a number of extracellular local concentrations are known over time. Optimally, the development of the extracellular local concentrations is determined essentially continuously over time.

As used herein, the term "neuroactive compound" may be understood in the broadest sense as any molecule that may provoke a pharmacologic and/or toxicologic effect on at least one neurocircuitry in a subject. The neuroactive compound may be a small-molecular neuroactive compound of not more than 500 Da in weight or may be a high-molecular weight neuroactive compound of more than 500 Da in weight. When administered to a subject of interest, the neuroactive compound may, optionally, be the plain neuroactive compound, a pharmaceutically acceptable salt thereof or a conjugated form thereof (e.g., conjugated to a pharmaceutically acceptable molecule).

In particular when the neuronal region is located in the brain, the neuroactive compound may be either pass the blood-brain-barrier by its own or may be conjugated to molecular structures facilitation uptake (e.g., hydrophilic residues may be capped (e.g., by means of acetylating or acylating hydroxyl and/or amino residues), the neuroactive compounds may be provided in liposomes and/or polymersomes, the neuroactive compound may be conjugated to a molecular structure recognized by a molecular transporter and/or the neuroactive compound may be conjugated to cell-penetrating peptide(s) (e.g, polyarginine etc.)). The neuroactive compound may be a molecule inherently present in the subject's body (e.g., a hormone or another signalling molecule) or may be a xenobiotic, i.e., a molecule typically not present in the subject's body. Preferably, the neuroactive compound is a xenobiotic.

When the neuroactive compound is pharmacologically active, it may also be designated as "drug", "agent", "medicament", "pharmacon", "medicine" or "pharmaceutical". When the neuroactive compound is toxicologically active, it may also be designated as "toxine" or "venom". The neuroactive compound may also be a "psychoactive", "neuropsychoactive", "neuromodulative" and/or "neuroactive" compound.

The one or more neuroactive compound(s) may be administered in different administration schemes such as, e.g., acute (e.g., as a bolus administration) and/or chronically (e.g., for at least few days, for at least a week or for at least one or more months or even years). Neurocircuitries may, optionally, differ for neuroactive compound(s) upon their route of administration even of the same neuroactive compound. Likewise, upon different routes of administration such as, e.g., systemic administration comprising the first-pass effect (e.g., orally), systemic administration bypassing the first-pass effect (e.g., intravenously (*i.v.*), intraperitoneally (*i.p.*), subcutaneously (*s.c.*), percutaneously, nasally, respiratorilly) or local administration (e.g., via injection into a neuronal region, into the spinal marrow, into the cerebrospinal fluid, into a nervous strand and/or into a synapsis), neurocircuitries may, optionally, differ even of the same neuroactive compound. Moreover, dependent on the administered dose (e.g., indicated as mg/kg) of a neuroactive compound, neurocircuitries may be influenced in different ways even of the same neuroactive compound.

The activity of each neurotransmitter may also be influenced by one or more pharmacologically and/or toxicologically active neuroactive compound(s). A neuroactive compound may interact selectively, i.e., it may have a preference for a particular neurotransmitter receptor subtype or even a preference or a particular isotype of such receptor. Alternatively, it may be rather unspecifically, i.e., binding to different subtypes and isotypes of a neurotransmitter receptor subtype. Thereby, a neuroactive compound may influence neurochemical connectivity patterns (neurocircuitries). A neuroactive compound may act as an agonist or an antagonist or an ambivalent agonist/antagonist. The person skilled in the art will know that the pharmacologic and/or toxicologic effect of a neuroactive compound may depend on the amount of neuroactive compound administered to a subject (i.e., may be dose-dependent) and the route of administration.

As used herein, the term "agonist" may be understood in the broadest sense as any neuroactive compound that increases the neurotransmitter activity. As used herein, an increase in the neurotransmitter activity may be an increase by at least 10%, at least 20%, at least 30%, at least 50%, at least 70%, at least 100%, at least 150%, at least 200% or even more compared to the comparable system without the presence of an agonist. Herein, the activity may, exemplarily, be an increase in the extracellular local concentration of the neurotransmitter of interest. Alternatively or additionally, the activity may also be blood oxygen level in a brain region.

Alternatively or additionally, the activity may also be electrical activity pattern in a brain region (e.g. EEG pattern, local field potential, etc.). Alternatively or additionally, the activity may also be the alteration in gene transcription level and/or rate of tissue. Alternatively or additionally, the activity may also be the alteration of the action potential of a cell bearing one or more receptors specific for the neurotransmitter of interest located in contact with the extracellular space observed. Alternatively or additionally, the activity may also be the alteration of cell or tissue behavior (e.g., contraction pattern) and/or may be even the macroscopic alteration of a systemic animal, including human (e.g., movement pattern, craving pattern, occurrence of withdrawal symptoms, etc.). An agonist may be a neuroactive compound present in the subject of interest inherently (e.g., a hormone, a neurotransmitter or another signalling molecule) or may be a xenobiotic, i.e., a neuroactive compound administered to said subject (e.g., a pharmacologically active and/or toxic neuroactive compound). As used throughout the present invention, a neurotransmitter receptor may be an ionotropic (i.e., be an ion channel opened or closed by one or more neurotransmitter(s)) or a metabotropic receptor (i.e., a receptor triggering intracellular signal transduction (e.g., a G-protein coupled receptor)).

An agonist may, exemplarily, interact with the respective neurotransmitter binding site(s) of one or more neurotransmitter receptor(s) wherein it mimicks the neurotransmitter and thereby provokes a cellular response similar to or even equal to the action of the neurotransmitter. Nicotine and muscarine, for instance, may trigger each acetylcholine receptors. Apomorphine and bromocriptine, for instance, may trigger each dopamine receptors. Clonidine, for instance, may trigger norepinephrine receptors. Buspirone, sumatrptane and lysergic acid diethylamide, for instance, may trigger serotonin receptors. Muscimol and Baclufene, for instance, may trigger gamma-aminobutyrate receptors. Opioid receptor agonists may be morphine, heroin, hydrocodone, oxycodone, codeine and/or methadone.

Alternatively or additionally, the agonist may interact with one or more respective neurotransmitter receptor(s) allosterically, i.e., by interacting with said receptor(s) at a site beyond the neurotransmitter binding site(s) and thereby provokes a cellular response similar to or even equal to the action of the neurotransmitter. Benzodiazepines (e.g., diazepam), barbiturates (e.g., pentobarbital) and ethanol, for instance, may trigger gamma-aminobutyrate receptors allosterically. Ethanol, for instance, may further potentiate the activity of acetylcholine receptors and serotonin receptors.

Alternatively or additionally, the agonist may interact with one or more factors (most typically proteins) associated in the intracellular signal transduction process(es) and thereby provokes a cellular response similar to or even equal to the action of the neurotransmitter. Alternatively or additionally, the agonist may increase the release level and/or rate of the neurotransmitter. Latrotoxines (e.g., $\alpha$-latrotoxine), for instance, may cause the rapid release of acetylcholine from intracellular vesicles and thereby causes strong activity of the acetylcholine pathway.

Alternatively or additionally, the agonist may decrease the resorption level and/or rate of the neurotransmitter. Cocaine and despramine, for instance, may inhibit norepinephrine transporters, thereby prevent norepinephrine re-uptake and indirectly increase the extracellular norepinephrine concentration and action thereof. Selective serotonin re-uptake inhibitors (SSRIs) (e.g., fluoxetine, prozac) and clomipramine may inhibit serotonin transporters, thereby prevent norepinephrine re-uptake and indirectly increase the extracellular norepinephrine concentration and action thereof.

Alternatively or additionally, the agonist may increase the provision level and/or rate of the neurotransmitter or a precursor of said neurotransmitter (e.g., the transport and/or synthesis thereof) or may be a precursor itself. DOPA, for instance, may be administered as a precursor for dopamine and, thereby, may increase the extracellular local concentration of dopamine, norepinephrine and/or adrenaline.

Alternatively or additionally, the agonist may decrease the degradation rate of the neurotransmitter. Organophosphates, for instance, may inhibit the acetylcholine esterase and thereby prevent degradation of acetylcholine, whereby the extracellular local concentration increases. Deprenyl may, exemplarily, inhibit monoamine oxidase(s) (MAO(s)) and may, thereby, increase the extracellular local concentration of dopamine.

As used herein, the term "antagonist" may be understood in the broadest sense as any neuroactive compound that decreases the neurotransmitter activity. As used herein, a decrease in the neurotransmitter activity may be a decrease to not more than 90%, not more than 80%, not more than 70%, not more than 60%, not more than 50%, not more than 40%, not more than 30%, not more than 20%, not more than 10%, not more than 5%, not more than 1% or even less compared to the comparable system without the presence of an antagonist. Herein, the activity may, exemplarily, be an increase in the extracellular local concentration of the neurotransmitter of interest. Alternatively or additionally, the activity may also be blood oxygen level in a brain region. Alternatively or additionally, the activity may also be electrical activity pattern in a brain region (e.g. EEG pattern, local field potential, etc.).

Alternatively or additionally, the activity may also be the alteration in gene transcription level and/or rate of tissue. Alternatively or additionally, the activity may also be the alteration of the action potential of a cell bearing one or more receptors specific for the neurotransmitter of interest located in contact with the extracellular space observed. Alternatively or additionally, the activity may also be the alteration of cell or tissue behavior (e.g., contraction pattern) and/or may be even the macroscopic alteration of a systemic animal, including human (e.g., movement pattern, craving pattern, occurrence of withdrawal symptoms, etc.). An antagonist may be a neuroactive compound present in the subject of interest inherently (e.g., a hormone, a neurotransmitter or another signalling molecule) or may be a xenobiotic, i.e., a neuroactive compound administered to said subject (e.g., a pharmacologically active and/or toxic neuroactive compound).

An antagonist may, exemplarily, interact with the respective neurotransmitter binding site(s) of one or more neurotransmitter receptor(s) wherein it partly mimicks the neurotransmitter and blocks the receptor and thereby inhibits a cellular response of a neurotransmitter. Atropine, curare, pirenzepin, atracurium, $\alpha$-bungarotoxine, $\alpha$-neurotoxine and hexamethonium, for instance, may inhibit each one or more type(s) of acetylcholine receptors. Neuroleptica such as, e.g., haloperidol and clozapine, for instance, may inhibit each dopamine receptors. Phentolamine and prazosine, for instance, may inhibit norepinephrine receptors. Ondansetrone and methylsergide, for instance, may inhibit serotonin receptors. Diphenhydramine and cimetidine, for instance, may inhibit histamine receptors. Bicuculline, for instance, may inhibit gamma-aminobutyrate receptors.

Alternatively or additionally, the antagonist may interact with one or more respective neurotransmitter receptor(s) allosterically, i.e., by interacting with said receptor(s) at a site beyond the neurotransmitter binding site(s) and prevents the interaction between the neurotransmitter receptor(s) with the neurotransmitter and thereby inhibits a cellular response of the neurotransmitter.

The drug Picrotoxine, for instance, may inhibit gamma-aminobutyrate receptors allosterically. Ethanol, for instance, may inhibit glutamate receptors.

Alternatively or additionally, the antagonist may interact with one or more factors (most typically proteins) associated in the intracellular signal transduction process(es) and thereby inhibits a cellular response of the neurotransmitter. Methylxanthines (e.g. theophylline, caffeine and theobromine) may influence the function of signal transduction pathways mediated by G-protein coupled receptors.

Alternatively or additionally, the antagonist may decrease the release level and/or rate of the neurotransmitter. Botulinus toxin A, for instance, inhibits the release of neurotransmitters such as, e.g., acetylcholine, by inhibiting the exocytosis thereof.

Alternatively or additionally, the antagonist may increase the resorption level and/or rate of the neurotransmitter.

Alternatively or additionally, the antagonist may decrease the provision level and/or rate of the neurotransmitter or a precursor of said neurotransmitter (e.g., the transport and/or synthesis thereof). Hemicholinium-3, for instance, may reduce the provision of choline to the axons and, thereby, lowers the production level and rate of acetylcholine. Alpha-methyl-p-tyrosine (AMPT), for instance, may prevent the conversion of tyrosine to DOPA and may, thereby, lower the extracellular local concentration of DOPA, dopamine, norepinephrine and/or adrenaline. Reserpine may, exemplarily, prevent dopamine storage within vesicles and may, thereby, lower the extracellular local concentration of dopamine.

Alternatively or additionally, the agonist may increase the degradation rate of the neurotransmitter.

Several neuroactive compounds may also be ambivalent agonists/antagonists. Exemplarily, such ambivalent agonists/antagonists may serve as agonists in one tissue and as antagonists in another tissue what may, exemplarily, be due to their different action on different receptor types. Furthermore, such ambivalent agonists/antagonists may, exemplarily, also serve as agonists and antagonists at different time points, what may be caused by the adaptation of the systems to the permanent presence of a neuroactive compound. Exemplarily, ethanol may have different effects dependent on the administered amount.

Whereas it may even be motivating and increasing activity in low amounts, higher amounts typically lead to tiredness and less activity. Chronical use of cocaine may alter the number of epinephrine transporters and ethanol may alter the number of gamma-aminobytryrate receptors. Then, the activity pattern may further alter.

It may further be noted that also other one or more neurotransmitter(s) may serve as agonist(s) or antagonist(s) in the sense of the present invention, when an influence on the sensitivity of the action mediated by another type of neurotransmitter is enabled. Exemplarily, ATP may influence the signal mediated by noradrenaline and/or acetylcholine. Ethanol, for instance, may enhance the activity of gamma-aminobutyrate receptors.

Also, a molecule inherently present in the subject of interest (e.g., a hormone or a combination of two or more thereof, a neurotransmitter or a combination of two or more thereof, another signalling molecule or a combination of two or more thereof or a combination of two or more of the aforementioned) may optionally influence the activity of a neurotransmitter in the sense of the present invention, i.e., serve as an agonist, antagonist or ambivalent agonist/antagonist. Exemplarily, a eicosanoid (e.g., a prostaglandin, a leukotriene, a thromboxane and/or a prostacycline) may provoke activity of several neurotransmitters associated with conveying a pain perception. Some molecules which can also serve as neurotransmitters may also have other functions in a body which may, optionally, influence neurocircuitries. Exemplarily, adrenaline and norepinephrine may also serve as hormones which may be systemically released and, thereby, also influence neurocircuitries.

As used herein, the term "evolution of" may be understood in the broadest sense as development over time. Therefore the evolution of extracellular local concentrations of the one or more neurotransmitter(s), for instance, refers to the alterations of said concentrations over time, therefore the time-resolved progression of the concentration profile. The evolution of extracellular local concentrations may be qualitative evolution.

An initial concentration as used herein is such concentration present at the beginning of observance time. Therefore, the initial concentration of a neuroactive compound may be understood as the concentration of a neuroactive compound at the beginning of observance time.

Additionally or alternatively, the initial concentration of a neuroactive compound may be understood as the maximal concentration. The person skilled in the art will know that the concentration of a xenobiotic neuroactive compound will typically approximately follow Bateman kinetics. Therefore, the may be an initial increase in concentration followed by a decrease. The velocity of increase may depend on the route of administration, the bioavailability, the hydrophobicity, the molecular weight, the plasma bound fraction and/or whether there are any transporters suitable for said neuroactive compound or precursor thereof. The degradation may typically comprise one or more enzymatic steps such as, e.g., oxidation, reduction, conjugation and/or degradation step(s) followed by elimination via the urinal tract, the gastrointestinal tract, sweating, breastfeeding and/or breathing. All these kinetics are well-known to those skilled in the art and may be, optionally, included in the methods according to the present invention.

As indicated above, the present method provides neurocircuitry modelling, therefore, provision of a model of one or more neurocircuitry/neurocircuitries.

As used herein the term "basal extracellular local concentration" may be understood as the extracellular local concentration of a neurotransmitter at the non-triggered state of the neuron (typically the concentration present on the initial time point $t = 0$). The basal extracellular local concentration of a neurotransmitter $k$ in a neuronal region $l$ may also be indicated as $s_l^k(0)$.

As used herein the term "basal extracellular local concentration" may be understood as the extracellular local concentration of a neurotransmitter at the non-triggered state of the neuron (typically the concentration present on the initial time point $t = 0$) may be understood as basal extracellular local concentration of said neurotransmitter, i.e., the concentration provided by $s_l^k(0)$.

The subject herein is preferably a mammal, more preferably a rodent or a primate, even more preferably a rat, a mouse or a human, in particular a rat. Possible values for $s_l^k(0)$ are further exemplified in the example section below. The person skilled in the art will, however, notice that for each subject, the respective extracellular local concentrations may optionally be adjusted accordingly. The respective values may be obtained from former experiments or may be determined by any means known in the art such as, e.g., by microdialysis (in particular dual-probe), voltammetry, proteomics, magnetic resonance tomography (MRT), pharmacological MRT based on local cerebral blood perfusion, positron emission tomography (PET), single-photon emission computed tomography (SPECT) and/or other biosensors. The basal extracellular local concentration level as well as the pattern of concentration alterations may then be higher or lower than a basal extracellular local concentration level of a comparable system without the respective neuroactive compound(s).

Typically, also after the regeneration of a neuronal system is completed the basal extracellular local concentration is achieved, which may with respect to a neurotransmitter k in a neuronal region $l$ also be indicated as $s_l^k(\tau_\alpha)$. Therefore, typically $s_l^k(0) \approx s_l^k(\tau_\alpha)$. The basal extracellular local concentration of a neurotransmitter k in a neuronal region $l$ $(s_l^k(0))$ may be the concentration found on average for said neurotransmitter $k$ in neuronal region $l$ throughout a population (i.e., may be a standardized value (e.g., derived from formerly determined dosimetry and physiologically-based pharmacokinetics)) or may be determined by experimentation (i.e., measurement (e.g., by one or more *in vivo* measurement(s))). Both routes provide on experimentally founded data.

The initial functions for each variable $s_l^k(t)$ may be generated as continuous and differentiable interpolation of a series of values $s_i^k(0)+\varepsilon$ within the interval $[\tau\alpha,0]$, with $\varepsilon$ representing a very small perturbation within the standard deviation of the average values.

Preferably, the basal extracellular local concentration of a neurotransmitter is the concentration present in the neuronal region when the neurotransmitter system is not excited, i.e. merely shows basic activity. More preferably, the basal extracellular local concentration of a neurotransmitter is the concentration present in the neuronal region when the neurotransmitter system is neither excited nor influenced by any influences beyond the physiological state. Particularly preferably, the basal extracellular local concentration of a neurotransmitter is the neurotransmitter concentration averagely present in the neuronal region throughout a population of subjects wherein said neurotransmitter system is not excited (i.e., repeatedly returned to basal conditions).

Alternatively, the basal extracellular local concentration of a neurotransmitter may also be increased or decreased upon non-physiologic influences such as, e.g., one or more pharmacologically and/or toxicologically neuroactive compounds, in particular when administered to a subject for a longer time, and/or one or more pathologically heath(s) conditions associated with neurocircuitries of the subject, in particular when said pathologically heaths condition(s) are chronic pathologically heaths condition(s). Typically, the basal extracellular local concentration of a neurotransmitter is in the (sub)nano-molar or the lower micromolar range.

Exemplarily, the basal extracellular local concentration $s_i^k(0)$ of glutamate (Glu) $\left(s_i^{Glu}(0)\right)$ in the brain may be in the range of between 50 nM and 5 $\mu$M depending on the brain region. In more detail, $s_i^{Glu}(0)$ may be approximately 4.5 $\mu$M in Amy, approximately 4.3 $\mu$M in Hb, , approximately 3.9 $\mu$M in OB and S, approximately 2.6 $\mu$M in Hc, approximately 2.4 $\mu$M in LC, approximately 2.1 $\mu$M in Acb, approximately 1.2 $\mu$M in Raphe, PFC, Ins and/or Hyt, approximately 1.0 $\mu$M in CPu, approximately 0.8 $\mu$M in BST and/or Th, approximately 0.43 $\mu$M in GP, approximately 0.21 $\mu$M in VTA, approximately 0.14 $\mu$M in SN, approximately 0.12 $\mu$M in STh and/or approximately 0.08 $\mu$M in Pn.

Exemplarily, the basal extracellular local concentration $s_i^k(0)$ of norepinephrine (noradrenaline, NA) $\left(s_i^{NA}(0)\right)$ in the brain may be in the range of between 0.4 nM and 16 nM depending on the brain region. In more detail, $s_i^{NA}(0)$ may be approximately 15.1 nM in Pn, 2.4 nM in BST and/or LC, approximately 2.3 nM in OB, approximately 1.4 nM in Th, approximately 1.2 nM in CPu, approximately 1.1 nM in PFC, Ins and/or S, approximately 0.6 nM in Amy, approximately 0.5 nM in Hyt and Hb and/or approximately 0.4 nM in Hc.

Exemplarily, the basal extracellular local concentration $s_i^k(0)$ of serotonin (5-hydroxytryptamine, 5-HT) $\left(s_i^{5-HT}(0)\right)$ in the brain may be in the range of between 0.1 nM and 2.5 nM depending on the brain region. In more detail, $s_i^{5-HT}(0)$ may be approximately 2.2 nM in Acb, approximately 0.9 nM in Amy and/or Raphe, approximately 0.8 nM in PFC, approximately 0.3 nM in Ins, BST, Hyt, Hb, Hc, Th,, SN and/or VTA and/or approximately 0.2 nM in OB and/or LC.

Exemplarily, the basal extracellular local concentration $s_i^k(0)$ acetylcholine (ACh) $\left(s_i^{ACh}(0)\right)$ in the brain may be in the range of between 0.4 nM and 150 nM depending on the brain region. In more detail, $s_i^{ACh}(0)$ may be approximately 140 nM in CPu, approximately 40 nM in HyT and/or Hc, approximately 35 nM in Acb, approximately 30 nM in Amy, Hb and/or PFC, approximately 15 nM in PN, approximately 13 nM in S, approximately 5.7 nM in VTA, approximately 4.1 nM in SN, approximately 3.5 nM in Raphe and/or approximately 0.4 nM in Th.

Exemplarily, the basal extracellular local concentration $s_i^k(0)$ of dopamine (DA) $\left(s_i^{DA}(0)\right)$ in the brain may be in the range of between 0.05 nM and 6 nM depending on the brain region. In more detail, $s_i^{DA}(0)$ may be approximately 5.8 nM in Raphe, approximately 5.1 nM in CPu, approximately 4.4 nM in Acb, approximately 2.0 nM in Ins, approximately 1.0 nM in Amy, approximately 0.9 nM in S, approximately 0.8 nM in BST, approximately 0.7 nM in VTA, approximately 0.5 nM in PFC, approximately 0.4 nM in LC, approximately 0.3 nM in Hb and/or SN, approximately 0.2 nM in Hc and/or Th and/or approximately 0.1 nM in STh.

Exemplarily, the basal extracellular local concentration $s_l^k(0)$ of gamma-aminobutyrate (GABA) $(s_l^{GABA}(0))$ in the brain may be in the range of between 8 nM and 0.7 $\mu$M depending on the brain region. In more detail, $s_l^{GABA}(0)$ may be approximately 0.64 $\mu$M in S, approximately 0.23 $\mu$M in Th, approximately 0.18 $\mu$M in Hb, approximately 0.11 $\mu$M in BST, approximately 0.10 $\mu$M in Hc, approximately 0.09 $\mu$M in Acb and/or Pn, approximately 0.06 $\mu$M in Amy, approximately 29 nM in HyT, approximately 21 nM in GP, approximately 18 nM in SN, approximately 17 nM in CPu, approximately 16 nM in VTA and/or approximately 9 nM in STh.

As used herein, the term "averaged basal extracellular local concentration" may be understood as the basal extracellular local concentration (i.e. the extracellular local concentration in the non-triggered state) on average found in the neuronal region in the subject over time. The person skilled in the art will know that typically the extracellular local concentration typically fluctuates upon repeatedly alternating stimulations and rest states. The average herein refers to the averaged concentrations of the rest states. Preferably, the averaged basal extracellular local concentration may be understood as the basal extracellular local concentration found in a particular type of neuronal region throughout the population of healthy subjects of the same species.

The step (iv) of rescaling the simulated evolution of extracellular local concentrations of the one or more neurotransmitter(s) may be based on the weight parameters herein indicated as $\omega$ (for details see below). These weight parameters may be assessed from the basal extracellular local concentrations determined in a subject of interest, wherein the determination may be performed by any means known in the art (see above). Further, additionally or alternatively, also other neuroactive compound- and/or neurotransmitter-specific parameters may be adapted to rescale the simulated evolution of extracellular local concentrations of the one or more neurotransmitter(s).

The method according to the present invention simulates the development of concentrations over time (simulated evolution).

The method may be conducted by any means suitable for that purpose known in the art.

In a preferred embodiment, the neurocircuitry comprises a set of coupled differential equations, the coupling reflecting at least interactions of the one or more neurotransmitters between one neuronal region and another neuronal region.

In a more preferred embodiment, the number of coupled differential equations is proportional to the number of neuronal regions in the set of neuronal regions and/or proportional to the number of neurotransmitters.

Herein, the differential equations are preferably first order differential equations, i.e. the change in the concentration of the neurotransmitter is any function F depending on the concentration of the neurotransmitter

$$ds_l^k(t)/dt \;=\; F(s_l^k(t)),$$ whereby $l$ denotes a neuronal region and $k$ denotes a neurotransmitter and $t$ the time.

In the view of the above, the person skilled in the art may note that the method according to the present invention may comprise a combination of mass conservation law and neurocircuitries, represented by a structurally stable system of coupled non-linear delay differential equations, which may determine the changes of neurochemical concentrations as a function of connectivity-based local physiological activity.

The neurocircuitry represented by the method according to the present invention may also reflect physiological processes that may influence the extracellular local concentration of a neurotransmitter. Exemplarily, these processes may reflect the release of a neurotransmitter into the extracellular space in a neuronal region of interest, the resorption of the neurotransmitter once released, the afferent projection of the neurotransmitter and/or the regeneration of the neurotransmitter.

Accordingly, in a preferred embodiment, the neurocircuitry comprises a term reflecting release, the term reflecting release, for a determined neurotransmitter in a determined neuronal region, being correlative to the extracellular local concentration of said determined neurotransmitter in said determined neuronal region.

As used herein, from a physiological point of view, the release of a neurotransmitter may be understood in the broadest sense as any input of a neurotransmitter into the extracellular cellular space of interest. Herein, the terms "input", "feed", "entry" and "intake" may be understood interchangeably.

The term reflecting release as used herein may preferably be an additive term in the differential equations

$$ds_l^k(t)/dt = f_{lm}^k(t)s_l^k(t) + \ldots,$$ wherein $f_{lm}^k(t)$ is a correlating factor reflecting dependence on the original neuronal region $l$, the neurotransmitter $k$, the time $t$ and the target neuronal region $m$.

The dependence on the target neuronal region $m$ herein may be reflected by summing over the degrees of outgoing projections, i.e. $ds_l^k(t)/dt = \sum f_{lm}^k(t)s_l^k(t) + \ldots$

The release activity at a neuronal region (exemplarily indicated as $l$) may be influenced by one or more excitatory afferent(s) and/or by one or more inhibitory afferent(s). Preferably, the release for the region $l$ of interest may be expressed by the formula $F_l(t)=A_l(t)-B_l(t)$, wherein the excitatory afferent may be expressed by

$$A_l(t) := \frac{1}{Ex(l)} \sum_{j=1}^{Ex(l)} \omega^k \frac{s_j^k(t) - s_j^k(0)}{s_j^k(0)}$$

and the inhibitory afferent may be expressed by

$$B_l(t) := \frac{1}{Inh(l)} \sum_{p=1}^{Inh(l)} \omega^q \frac{s_p^q(t) - s_p^q(0)}{s_p^q(0)} \quad ,$$

wherein $k$ and $q$ each represent the neurotransmitter utilized for the neuronal projection towards region $l$.

Optionally, chronic drug exposure may alter the release rate and/or release kinetics. This is included by the weight parameters herein indicated as $\omega$ ($\omega_k$ for neurotransmitter $k$ and $\omega_q$ for neurotransmitter $q$). Whereas in a physiological state the weight parameters may be, preferably, set to 1 (i.e., $\omega_k = \omega_q = 1$), the parameters may be adapted to other values upon chronic drug exposure.

These weight parameters $\omega$ ($\omega_k$ and $\omega_q$), may be used for rescaling the simulated evolution of extracellular local concentrations of the one or more neurotransmitter(s) according to step (iv) of the present method (see above).

In a system of rest at $t = 0$, $F_l(t)$ preferably represents the neurochemical basal activity at the neuronal region $l$, i.e., $F_l(0) = 0$. Therefore, the system is normalized to no activity. The person skilled in the art will notice that in some regions there may optionally also be a low level basal release occurring without electrical stimulation. This may optionally also included into the method according to the present invention, e.g., bay means of a further function similar to the aforementioned with a random parameter to reflect additional random activations.

The function $F_l(t)$ may be represented by the saturation function (activity function)

$$\Gamma_l(t) \in [0, 1] \quad \text{with} \quad \Gamma_l(t) = 1 - \frac{1}{e^{4F(t)} + 1}$$

which may, similar to hysteresis-like operators, act as a switch for neurotransmitter dynamics. In the view of the functional relationships laid out above, it will be noted that a neuronal region $l$ may be activated when the excitatory afferents prevail over inhibitory afferents, whereas it may be deactivated when inhibitory afferents prevail.

In a neurocircuitry, the release functions may be sigmoidal. Herein, a real-valued parameter representing the averaging limit of the amplitudes of such sigmoidal release function induced by changes in normalized membrane potentials, may be represented by $a_{lm}^k > 0$. This may represent the ratio of the maximally released neurotransmitters in the neuronal region with respect to the total number of neurotransmitters in said region, which may be estimated by the non-linear initial function

$$\sum_{\substack{m=1 \\ m \neq l}}^{\deg^{out}(l)} a_{lm}^k$$

and the non-linear operator $f_{lm}^k(t) = a_{lm}^k \Gamma_l(t)$ representing the utilization of neurotransmitter $k$ at the neuronal region $l$ for projections towards target region $m$. Therefore, in the neurocircuitry of interest, the function representing the release of neurotransmitter $k$ towards target region $m$ may be

$$\sum_{\substack{m=1 \\ m \neq l}}^{\overline{deg^{out}(l)}} -f_{lm}^{k}(t) s_l^{k}(t)$$

This function comprises neurocircuitry topology (i.e., neuronal interconnection between regions $l$ and $m$) and functional neurochemistry (i.e., excitatory and inhibitory projections) therein.

Therefore, in a more preferred embodiment, the term reflecting release comprises a correlating factor being itself proportional to a saturation function.

According to the present invention, the number of incoming projections (i.e., afferents) to a region may be also understood as the in-degree $deg^{in}(l)$ that is composed of the addition of the number of excitatory afferents ($Ex(l)$) and the number of inhibitory afferents ($Inh(l)$) (i.e., $deg^{in}(l) = (Ex(l)) + (Inh(l))$. As used herein, such afferents may also be designated as "inputs" or "projections". Analogously, the number of outgoing projections from a region may be understood as the out-degree $deg^{out}(l)$. Accordingly, release of a neurotransmitter may influence the neuronal region in either an inhibitory or excitatory way. The neuronal region may be part of a neurocircuitry and if the total of excitatory influences herein is greater than that of inhibitory influences, it will also be excited and thereby release neurotransmitter into the extracellular space and, exemplarily, may transmit the information to yet another neighboring region.

As used throughout the present invention, the term "afferent" may be understood in the broadest sense as any input signal (neuronal projection) led into the neuronal region and, typically, when a certain threshold of the signal strength is overcome, may enable to activate the neuronal region and, thereby, trigger said neuronal region to release the neurotransmitter into the extracellular space. When the neuronal region is located in the central nervous system (CNS) such as, e.g., in the brain, the afferent may also optionally be an afferent neuron (also known as sensory neuron, receptor neuron or afferent axon) that enables the conveyance of an impulse from the periphery into the CNS and/or an impulse from one or more interconnected structure(s) in a neurocircuitry. The opposite of an afferent is an efferent, i.e., an output signal (neuronal projection) out of the neuronal region and, typically, conveyed into one or more other region(s), preferably conveyed from a region located in the CNS.

Exemplarily, the release of a neurotransmitter may be the exocytosis of a neurotransmitter, i.e., a process including the fusion of neurotransmitter-containing intracellular vesicles with the plasma membrane. Optionally, excocytosis may include the steps of vesicle trafficking, vesicle tethering, vesicle docking, vesicle priming and vesicle fusion. Optionally, the vesicles are coated vesicles (e.g., clathrin-coated vesicles). Exemplarily, the neurotransmitters acetylcholine, dopamine, norepinephrine, serotonin, glutamate, gamma-aminobutyrate, glycine, adenosine triphosphate are each typically, at least partly, released by means of excocytosis. Optionally, release may follow anterograde transport of the neurotransmitter and/or metabolic precursors thereof to the cell surface adjacent to the extracellular space of interest (e.g., the axon terminal on the presynaptic side of synapse).

Alternatively or additionally, the release may be or include neurotransmitter transport through the plasma membrane, optionally, mediated by one or more neurotransmitter transporter(s) (e.g., glutamate/aspartate transporter(s) (e.g., excitatory amino acid transporter 1 (EAAT1), excitatory amino acid transporter 2 (EAAT2), excitatory amino acid transporter 3 (EAAT3), excitatory amino acid transporter 4 (EAAT4), excitatory amino acid transporter 5 (EAAT5), vesicular glutamate transporter 1 (VGLUT1), vesicular glutamate transporter 2 (VGLUT2), vesicular glutamate transporter 3 (VGLUT3)), GABA transporter(s) (e.g., GABA transporter type 1 (GAT1), GABA transporter type 2 (GAT2), GABA transporter type 3 (GAT3), betaine transporter (BGT1), vesicular GABA transporter (VGAT)), glycine transporter(s) (e.g., glycine transporter type 1 (GlyT1), glycine transporter type 2 (GlyT2)), monoamine transporter(s) (e.g., dopamine transporter (DAT), norepinephrine transporter (NET), serotonin transporter (SERT), vesicular monoamine transporter 1 (VMAT1), vesicular monoamine transporter 2 (VMAT2)), adenosine transporter(s) (e.g., equilibrative nucleoside transporter 1 (ENT1), equilibrative nucleoside transporter 2 (ENT2), equilibrative nucleoside transporter 3 (ENT3), equilibrative nucleoside transporter 4 (ENT4)), vesicular acetylcholine transporter (VAChT)). The person skilled in the art will notice that certain types of neurotransmitter transporters are typically associated with the particular neurotransmitter(s). Different isotypes of such neurotransmitter transporters may be associated with different tissues, cells and cell sides.

Preferably, release of the neurotransmitter may be triggered in response to a threshold action potential or graded electrical potential in the cell said neurotransmitter is stored. Alternatively or additionally, release may also be triggered by neuroactive compound(s) such as, e.g., latrotoxine.

Neurochemical connectivity may be characterized by using the non-dimensionalized system parameters $a_{lm}^{k}$ (simulation parameters, preferably $a_{lm}^{k} < 1$), for a region of origin $l$ and a target region m ($l,m \in \{1,...,[number\ of\ target\ regions]\}$) and neurotransmitter $k \in \{1,...,[number\ of\ neurotransmitters]\}$). Possible values for $a_{lm}^{k}$ are exemplified in the example section below.

In the context of the present invention, in a neurocircuitry of interest, preferably, the time interval of between the initial time point $t = 0$ and the completion of regeneration of the extracellular local concentration of the neurotransmitter

$t = \tau_\alpha$ may be determined (i.e., for time interval $[\tau_\alpha, 0]$). Herein, preferably, initial functions with $\Gamma_l(t) = 1$ and the sum

$$\sum_{\substack{m=1 \\ m\neq l}}^{\mathbf{deg^{out}(l)}} a_{lm}^{k} = 1$$

may be used. The non-dimensionalized system parameters $a_{lm}{}^k$ for neurocircuitries in the brain (i.e., wherein the neuronal regions are brain regions) are further exemplified in the experimental section below.

As laid out above, the neurocircuitry represented by the method according to the present invention may also reflect the resorption of the neurotransmitter once released.

Accordingly, in a preferred embodiment, the neurocircuitry comprises a term reflecting resorption, said term reflecting resorption, for a determined neurotransmitter in a determined neuronal region, being correlative to the extracellular local concentration of said determined neurotransmitter in a neuronal region different from said determined region at earlier times.

Herein, the determined neuronal region may also be understood and designated as neuronal region of origin (indicated as *l*) and the neuronal region different from the determined region may be understood and designated as target neuronal region (indicated as *m*).

As used herein, from a physiological point of view, the resorption of a neurotransmitter may be understood in the broadest sense as any withdrawal of a neurotransmitter from the extracellular cellular space of interest. Herein, the terms "withdrawal" and "removal" may be understood interchangeably.

The resorption may be, therefore, understood as factual loss of neurotransmitter from the extracellular location of interest. The factual loss of a neurotransmitter *k* at a neuronal region different from the determined region (target region) *m* may be expressed by the parameter $\mu^k{}_m$ as used herein.

The factual loss may comprise cellular uptake of the neurotransmitter, metabolism of the neurotransmitter and extra-synaptic diffusion processes of the neurotransmitter.

Summarized the resorption may be expressed by $r_l^k(t\text{-}\tau_{lm})s_m^k(t\text{-}\tau_{lm})$ , wherein

$$r_l^k(t\text{-}\tau_{lm}) = \mu_m^k \Gamma_l(t - \tau_{lm})$$ may represent the amount of neurotransmitter *k* resorbed at target region *m* after it has been released. Herein, $\tau_{lm}$ represents the time required for conveyance of a signal from the region of origin *l* to the target region *m* in order to release the neurotransmitter in said target region *m.*

Exemplarily, the resorption of a neurotransmitter may be the endocytosis of a neurotransmitter, i.e., a process including the engulfing neurotransmitters by means of the formation of neurotransmitter-containing intracellular vesicles, wherein the vesicle membrane is formed from the plasma membrane. Optionally, endocytosis may be clathrin-mediated endocytosis, caveolae-mediated endocytosis or macro- or micropinocytosis. Typically, the resorption by means of endocytosis includes the (non-covalent) interaction of the neurotransmitter with a receptor located at the plasma membrane mediating selective endocytosis. Optionally, resorption may be followed by degradation of the neurotransmitter and/or retrograde transport of the neurotransmitter and/or metabolic products thereof.

Alternatively or additionally, the resorption may be or include neurotransmitter transport through the plasma membrane, optionally, mediated by one or more neurotransmitter transporter(s) such as, e.g., one or more of those laid out in the context of release above. Exemplarily, the neurotransmitters dopamine, norepinephrine, serotonin, glutamate, gamma-aminobutyrate, glycine, adenosine triphosphate and the neurotransmitter metabolite choline are each typically, at least partly, resorbed by means of one or more specific transporter(s). Different isotypes of such neurotransmitter transporters may be associated with different tissues, cells and cell sides.

Optionally, in order to overcome the under-determinedness of the system with respect to the system parameters, it may be assumed that $\forall m, \mu_m^k = \mu^k$ , i.e., that the metabolism may differ with respect to different neurotransmitters but may be essentially equal for a particular neurotransmitter in different regions. Based on this assumption, $\mu^k$ parameters may be estimated.

Typically, neurochemical systems in the brain may, in the active state, resorb between 50% and nearly 100%, preferably between 75% and nearly 100%, more preferably between 85% and 99%, even more preferably between 90% and 97% of the released neurotransmitters with metabolism parameters $\mu^k$ varying between 3-10% (e.g., with $\mu^{glu}$ of approximately 0.10, $\mu^{GABA}$ of approximately 0.06, $\mu^{ACh}$ of approximately 0.08, $\mu^{NA}$ of approximately 0.05, $\mu^{DA}$ of approximately 0.03 and/or $\mu^{5\text{-HT}}$ of approximately 0.04). To improve the computational properties of the system, numerical simulations may preferably be conducted on the non-dimensionalized system. For this purpose, the parameters $\mu^k$ of the non-dimensionalized system may, exemplarily, be for $\mu^{glu}$ approximately 1.56, for $\mu^{GABA}$ approximately 1.30, for $\mu^{ACh}$ approximately 0.07, for $\mu^{NA}$ approximately 0.50, for $\mu^{DA}$ approximately 0.02 and/or for $\mu^{5\text{-}HT}$ approximately 0.06. In the view of the aforementioned, the non-dimensionalized parameters do typically not fulfill the condition $0 < \mu_m^k, a_{lm}^k < 1$. The non-dimensionalized metabolism parameters $\mu^k$ are further exemplified in the experimental section below.

In the view of the above, summarized, it will be noted that, according to the method of the present invention, resorption is preferably correlative to the saturation function at an earlier time point. Preferably, it is summated over the number of (efferent) projections. Optionally, it may further also be summated over the earlier time points.

As laid out above, the neurocircuitry represented by the method according to the present invention may also reflect the afferent projections of the neurotransmitter.

Accordingly, in a preferred embodiment, the neurocircuitry comprises a term reflecting afferent projections, the term reflecting afferent projections, for a determined neurotransmitter in a determined neuronal region, being correlative to an amount of said determined neurotransmitter in a neuronal region different from the determined region at earlier times.

As in the context of resorption above, the determined neuronal region may also be understood and designated as neuronal region of origin (indicated as *l*) and the neuronal region different from the determined region may be understood and designated as target neuronal region (indicated as *m*).

Herein, it is preferably summated over the number of incoming projections.

According to the present invention, the afferent projections may also, from a physiological point of view, understood as entrance of the neurotransmitter *k* into the extracellular cellular space of the neuronal region of origin *l* from one or more neighboring region(s) *j* such as from adjacent or proximal region(s) such as, e.g., adjacent or proximal synapse(s). This may be expressed by the formula

$$\sum_{\substack{j=1 \\ j \neq l}}^{\deg^{in}(l)} f_{jl}^k(t-\tau_{jl})s_j^k(t-\tau_{jl})$$

Which represents the portion of the released neurotransmitter *k* from neighboring region(s) *j* that reaches the neuronal region *l*.

As laid out above, the neurocircuitry represented by the method according to the present invention may also reflect the regeneration of the neurotransmitter.

Accordingly, in a preferred embodiment, the neurocircuitry comprises a term reflecting regeneration of the one or more neurotransmitter(s).

As used herein, from a physiological point of view, the regeneration of the neurotransmitter balance may be understood in the broadest sense as any recuperation of the basal concentration level of the neurotransmitter. Herein, the terms "regeneration", "recuperation" and "restoration" may be understood interchangeably. The basal concentration level may be understood in the broadest sense as an extracellular local concentration of the neurotransmitter enabling subsequent further cellular actions. The process of regeneration typically leads to the maintenance of a basal concentration level of the neurotransmitter. This may include, on the one hand the providing of the neurotransmitter, exemplarily, by means of (biochemical) synthesis and/or by diffusion of the neurotransmitter into the neuronal region (also designatable addition of the neurotransmitter), on the other hand the degradation of the neurotransmitter and/or by diffusion of the neurotransmitter out of the neuronal region (also designatable as deficit or shortage of neurotransmitter).

Exemplarily, the provision of the neurotransmitter may include one or more biochemical process(es) leading to the reformation or even re-synthesis of the neurotransmitter from a precursor molecule. Exemplarily, acetylcholine may be provided by the enzymatically catalyzed acetylation of choline. Dopamine may, exemplarily, be provided by the enzymatically catalyzed hydroxylation of tyrosine to DOPA and the, likewise, enzymatically catalyzed decarboxylasis dihydroxyphenylalanine to dopamine. norepinephrine may, exemplarily, be provided by the enzymatically catalyzed hydroxylation of dopamine. Serotonin may, exemplarily, be provided by the enzymatically catalyzed hydroxylation of tryptophan.

Exemplarily, the degradation of the neurotransmitter may include one or more biochemical process(es) leading to the decomposition and/or inactivation of the neurotransmitter. Exemplarily, acetylcholine may be enzymatically cleaved into choline and acetate. Dopamine may, exemplarily, be metabolized by the enzymatically catalyzed methylation (to 3-methoxytyramine) and/or oxidation to the respective aldehyde. norepinephrine may, exemplarily, be metabolized by the enzymatically catalyzed methylation (to normetanephrine) and/or oxidation to the respective aldehyde. Serotonin may, exemplarily, be metabolized by the enzymatically catalyzed oxidation to the respective aldehyde. Typically, the degradation of the neurotransmitter leads to a less active or even inactive metabolite. This metabolite may be excited from the body, metabolized further or may be recycled to the neurotransmitter. Most metabolites are excited from the body and/or metabolized further. Some like choline, however, are recycled to the neurotransmitter like acetylcholine.

From a physiological point of view, the regeneration of the neurotransmitter balance may lead to a basal extracellular local concentration level in the absence of a stimulus. However, the chronic administration of a neuroactive compound or a combination of neuroactive compounds may also alter the basal extracellular local concentration level. Likewise, also some neuroactive compounds such as, e.g., anestetica (e.g., benzodiazepines) may have an influence on the basal extracellular local concentration level. Then, the basal extracellular local concentration level used in the method according to the present invention may preferably be adapted to the anesthesia-induced basal extracellular local concentration level.

Regeneration of the basal extracellular local concentration level as used herein may be expressed by the term

$$[s_l^k(t-\tau_\alpha)-s_l^k(t-2\tau_\alpha)]$$

representing the deficit in the extracellular local concentration of the neurotransmitter $k$ in the neuronal region $l$ after a cycle of information processing.

When herein the extracellular local concentration of the neurotransmitter $k$ in the neuronal region $l$ upon completion of a cycle of information processing is increased ($\tau_\alpha = max_{l \neq m}\tau_{lm}$), this term is > 0. Then, the step of regenerating the concentration is lowering the concentration such as, e.g., by means of degrading neurotransmitter, up-taking neurotransmitter and/or diffusing away thereof.

In contrast, the aforementioned term may be < 0 when the extracellular local concentration of the neurotransmitter $k$ in the neuronal region $l$ is increased upon completion of a cycle of information processing ($\tau_\alpha = max_{l \neq m}\tau_{lm}$). Then, the step of regenerating the concentration is increasing the concentration such as, e.g., by means of re-synthesis of the neurotransmitter, excreting the neurotransmitter and/or intake by diffusion into the neuronal region. Herein, the time delay $\tau_\alpha$ of in the vector fields typically differs from the time intervals of information processing pathways.

As used throughout the present invention, the terms "time delay" and "time lag" may be understood interchangeably in the broadest sense as the time, the signal conveyance requires from one neural region to the other. The time delays expressible as $\tau_{lm}$ may be understood as the time that is required for a signal to reach the target region $m$ from the region of origin $l$. It will be understood that, *vice versa,* when the time delay is $\tau_{ml}$, the target region is $l$ and the region of origin is $m$. As used herein, the time delays may preferably be assumed to be essentially symmetrical (i.e. $\tau_{lm} \approx \tau_{ml}$). Further, preferably a physically plausible linear relationship between the anatomical distances of projections and time delays (i.e, x = v$\tau$) may be assumed. Alternatively, when diffusional processes play a larger role, also a non-linear relationship may be assumed such as, e.g., an exponential function. Exemplarily, when the neuronal regions are brain regions, said brain regions may be assumed to be portioned in six clusters I-VI: cluster I (OB), cluster II (PFC, Ins, Acb, CPu, S), cluster III (BST, GP, HyT, Amy), cluster IV (Hb, Hc, Th, STh), cluster V (SN, VTA), and cluster VI (R, LC, Pons). Herein, the following time delays may be assumed:

> interaction between cluster I and cluster II: approximately 3 ms;
> interaction between cluster I and cluster III: approximately 4 ms;
> interaction between cluster I and cluster IV: approximately 5 ms;
> interaction between cluster I and cluster V: approximately 6 ms;
> interaction between cluster I and cluster VI: approximately 8 ms;
> interaction between cluster II and cluster III: approximately 2 ms;
> interaction between cluster II and cluster IV: approximately 3 ms;
> interaction between cluster II and cluster V: approximately 4 ms;
> interaction between cluster II and cluster VI: approximately 5 ms;
> interaction between cluster III and cluster IV: approximately 1 ms;
> interaction between cluster III and cluster V: approximately 2 ms;
> interaction between cluster III and cluster VI: approximately 4 ms;
> interaction between cluster IV and cluster V: approximately 1 ms;
> interaction between cluster IV and cluster VI: approximately 3 ms; and/or
> interaction between cluster V and cluster VI: approximately 2 ms.

The usable time delays in the context of brain regions are further exemplified in the example section below.

Moreover, as used herein, a neurocircuitry may comprise an intrinsic memory of the system, i.e., may define an anatomical memory based on the neurochemical connectomics. This may enable to determine altered basal extracellular concentrations of the neurotransmitter k (e.g., due to particular disease(s) and/or chronic administration of one or more neuroactive compound(s)) and to determine effects on a neurocircuitry and neurocircuitry patterns arising therefrom. Accordingly, the extracellular local concentration present after completion of a cycle of information processing ($\tau_\alpha = max_{l \neq m} \tau_{lm}$) may, after regeneration is completed, be approximately the same concentration as that at the initial time point $t = 0$. Therefore, typically, $s_l^k(0) \approx s_l^k(\tau_\alpha)$. This may enable a neuron to fulfill its entire function again after regeneration is completed. However, it may be noted that, optionally, the basal extracellular concentrations of the neurotransmitter $k$ may be altered over time, in particular upon the presence of disease(s) and/or chronic administration of one or more neuroactive compound(s). Additionally or alternatively, the basal extracellular concentrations of the neurotransmitter $k$ may optionally also be influenced by other excitatory and/or inhibitory neurotransmitter(s), signalling molecule(s), hormone(s) and/or xenobiotic neuroactive compound(s). Additionally or alternatively, a neuron may also be activated when the basal extracellular concentrations of the neurotransmitter $k$ is not entirely reached again, i.e., before regeneration has been completed. Then, typically, it may have a somewhat lower activity.

In the view of the above, preferably, the method according to the present invention may reflect at least two of the following physiologic processes as laid out above:

(a) the release of a neurotransmitter into the extracellular space in a neuronal region of interest;
(b) the resorption of the neurotransmitter once released;
(c) the afferent projection of the neurotransmitter; and/or
(d) the regeneration of the neurotransmitter,

more preferably wherein at least three of the above physiologic processes are reflected by the method according to the present invention, in particular wherein all the above physiologic processes (a)-(d) are reflected by the method according to the present invention.

Therefore, particularly preferably, the method according to the present invention may comprise reflecting a system of coupled non-linear delay-differential equations of the form

$$\frac{ds_l^k(t)}{dt} = \sum_{\substack{m=1 \\ m \neq l}}^{\deg^{out}(l)} -f_{lm}^k(t)s_l^k(t) + r_l^k(t-\tau_{lm})s_m^k(t-\tau_{lm}) + \sum_{\substack{j=1 \\ j \neq l}}^{\deg^{in}(l)} f_{jl}^k(t-\tau_{jl})s_j^k(t-\tau_{jl}) - [s_l^k(t-\tau_\alpha)-s_l^k(t-2\tau_\alpha)]$$

providing the dynamics of the extracellular local concentration $s_l^k(t)$ of the neurotransmitter $k$ in the neuronal region $l$ within a neurocircuitry (Noori and Jäger,

2010; Noori, 2012a,b; Noori et al., 2012a). The terms and abbreviations herein may be specified as laid out above.

As indicated above, the neurocircuitry represented by the method according to the present invention may also reflect the effect of one or more neuroactive compound(s) on the neurocircuitry.

In order to determine the impact of a neuroactive compound on the extracellular local concentration of the neurotransmitter $k$ in a neuronal region $l$ $\left(s_l^k(t)\right)$, first, the concentration of the neuroactive compound over time $c(t)$ present in the neuronal region may be determined. $c(t)$ may be determined by any means such as, e.g., determined experimentally *in vivo* (e.g., by forward dosimetry and/or physiologically-based pharmacokinetics such as pharmacologically-based pharmacokinetics (PBPK) modeling and/or may be determined by the relationship $c(t)=K_{p,u} P(t)$, wherein $K_{p,u}$ represents the ratio of unbound (active) neuroactive compound concentration in the neuronal region to its plasma concentration and $P(t)$ represents the blood neuroactive compound concentration over time.

Preferably, it may be assumed that the effect of a neuroactive compound on a neurocircuitry correlates with its active concentration in the neuronal region, the effect of said neuroactive compound is described by the term $\delta_{j,r}^k c_r^k(t)$, wherein $\delta_{j,r}^k$ represents the efficacy (potency) of the neuroactive compound per concentration with respect to the local concentration $s_j^k(t)$ of the neurotransmitter $k$ in the neuronal region $j$. Herein, $\delta_{j,r}^k$ may preferably embrace the sum of essentially all effects on the extracellular local concentrations of the neurotransmitter (including, exemplarily, direct and indirect agonistic and antagonistic effects). In general, $\delta_{j,r}{}^{k}$ may be a grey-box parameter determined by experimentation *in vivo*. Exemplarily, $\delta_{j,r}{}^{k}$ may be determined in one neuronal region and three different doses of the neuroactive compound in two different neuronal regions and two different doses of the neuroactive compound, and/or in two different neuronal regions with acute and chronic administration of a single dose of the neuroactive compound of interest.

To study the combinatorial effects of different neuroactive compounds simultaneously, the parameter $\delta_{j,r}{}^{k}$ may, preferably, be estimated in advance for each substance. Subsequently, the different terms may be integrated into the model components. The integration of the neuroactive compound-specific terms into the method according to the present invention is exemplified in detail in the example section.

The neuroactive compound may preferably be an agonist or an antagonist.

Accordingly, in a preferred embodiment, the neurocircuitry comprises a term reflecting the effect of one or more agonist(s) and/or antagonist(s) on the activity of the one or more neurotransmitter(s). Due to the fact that many neurotransmitters are functionally connected with another in neurocircuitries (cf., exemplarily, Noori et al. (2012), Addict Biol 17(5):827-864), the administration of one or more neuroactive compounds (e.g., agonist(s), antagonists(s), ambivalent agonist(s)/antagonist(s) or a combination of two or more thereof), may also influence the extracellular local concentrations of one or more other neurotransmitter(s) either directly (e.g., by triggering the excretion and/or exocytosis of the other neurotransmitter(s)) or indirectly (e.g., by changing the activity potential of a nervous strand leading to the transmission of an electrical current flow and finally the excretion and/or exocytosis of the other neurotransmitter(s)).

Accordingly, the administration of one or more neuroactive compounds may, optionally, alter the neurocircuitry pattern of a subject with respect to more than one neurotransmitter. This may, optionally, also provoke an alteration of complex behaviour patterns of the subject. Many examples are known. Exemplarily, the administration of ethanol provokes numerous alterations of human and animal behavior such as, e.g., slower reaction times, impairment of movement pattern, tiredness, etc.

As discusses in the context of release above, the interaction of different neurocircuitries and the activation of a neuronal region as the consequence of its neurochemical afferent(s) may be represented by the term $\Gamma_l(t) \in [0, 1]$ with

$$\Gamma_l(t) = 1 - \frac{1}{e^{4F(t)} + 1}$$

which may provide the neurotransmitter dynamics, wherein $F_l(t) = A_l(t) - B_l(t)$ represents the neurochemical balance function at region *l*. Herein, $A_l(t)$ represents the excitatory afferents and $B_l(t)$ represents the inhibitory afferents to the region *l*. Accordingly, the action of one or more agonist(s) and/or antagonist(s) *r* and *w* on the neurotransmitter systems *k* and *q*, respectively, may be represented by the neuroactive compound terms $\pm \sum_{r=1}^{n} \delta_{j,r}^{k} \, c_r^{k}(t)$ and $\pm \sum_{w=1}^{m} \delta_{n,w}^{q} \, c_w^{q}(t)$, wherein (+) typically indicates an agonism and (-) typically indicates an antagonism of the action of the respective neurotransmitter $s_j^{k}(t)$. Herein, the modified excitatory and inhibitory components of the activation process may be represented by the formulae

$$A_l(t) := \frac{1}{Ex(l)} \sum_{j=1}^{Ex(l)} \omega^k \frac{s_j^{k}(t) - s_j^{k}(0) \pm \sum_{r=1}^{n} \delta_{j,r}^{k} \, c_r^{k}(t)}{s_j^{k}(0)}$$

and

$$B_l(t) := \frac{1}{Inh(l)} \sum_{p=1}^{Inh(l)} \omega^q \frac{s_p^q(t) - s_p^q(0) \pm \sum_{w=1}^{m} \delta_{p,w}^q \, c_w^q(t)}{s_p^q(0)}$$ .

Agonists and antagonists may influence one or both of $A_l(t)$ and/or $B_l(t)$.

As indicated above, the extracellular local concentration of a neurotransmitter $k$ may be influenced directly or indirectly. Neuroactive compounds that bear a direct impact on the extracellular local concentrations of the neurotransmitter may be integrated into the vector fields of the main formulae as used herein by the term $\sigma^k(t) = \pm \sum_{h=1}^{N} \delta_{l,h}^k \, c_h^k(t)$. In result, this may result in the formula

$$\frac{ds_l^k(t)}{dt} = \sum_{\substack{m=1 \\ m \neq l}}^{deg^{out}(l)} -f_{lm}^k(t)s_l^k(t) + r_l^k(t-\tau_{lm})s_m^k(t-\tau_{lm}) + \sum_{\substack{j=1 \\ j \neq l}}^{deg^{in}(l)} f_{jl}^k(t-\tau_{jl})s_j^k(t-\tau_{jl}) - [s_l^k(t-\tau_\alpha) - s_l^k(t-2\tau_\alpha)] + \sigma^k(t)$$

As laid out above, the parameter $\delta_{l,h}^k$ may be a nonlinear grey-box parameter, which may be determined experimentally *in vivo* measuring the neuroactive compound-induced alterations of the neurotransmitter concentrations. Examples for such neuroactive compounds that bear a direct impact on the extracellular local concentrations of the neurotransmitter may be uptake inhibitors such as, e.g., selective serotonin re-uptake inhibitors (SSRIs).

Alternatively or additionally, a neuroactive compound or a combination of neuroactive compounds may have an impact on the basal extracellular local concentration of a neurotransmitter. In particular, anesthesia may bear such alterations of the basal extracellular local concentration of a neurotransmitter.

Accordingly, in a preferred embodiment, the neurocircuitry comprises a term reflecting the effect of anesthesia.

Such alterations due to anesthesia may influence the dynamical behavior of the neurochemical trajectories. Additionally or alternatively, such neuroactive compound(s) may interact with one or more other administered neuroactive compounds and thereby disturb the determinations for the neuroactive compound-induced effects.

Exemplarily, anesthesia may disturb the determinations for benzodiazepine-induced effects. This disturbance may, optionally, be overcome by means of experimentally measuring the effects of anesthesia on the neuroactive compound-induced effects, wherein the parameters $\delta_{j,r}^k$ for the anesthetic neuroactive compound and the neuroactive compound of interest are determined. Then, in the method according to the present invention, the basal extracellular local concentration of a neurotransmitter may be adjusted to the experimentally measured basal concentration.

As laid out above, a neuroactive compound in the context of the present invention may be any neuroactive compound. The neuronal region may be any neuronal region.

In a preferred embodiment, the neuroactive compound is a neuropsychoactive compound and/or the neuronal region is a brain region.

Even more preferably, the neuroactive compound is a neuropsychoactive compound and the neuronal region is a brain region.

As laid out above, the one or more neurotransmitter(s) in the context of the present invention may be any neurotransmitter(s).

In a preferred embodiment, at least one of the one or more neurotransmitter(s) is selected from the group consisting of glutamate, gamma-aminobutyrate, acetylcholine, dopamine, norepinephrine and serotonin.

More preferably, at least two neurotransmitters are object of the method according to the present invention, wherein said at least two neurotransmitters are even more preferably selected from the group consisting of glutamate, gamma-aminobutyrate, acetylcholine, dopamine, norepinephrine and serotonin.

Even more preferably, all neurotransmitter(s) that are object of the method according to the present invention are selected from the group consisting of glutamate, gamma-aminobutyrate, acetylcholine, dopamine, norepinephrine and serotonin, in particular when the neuronal regions l and m are both located in the central nervous system (CNS), in particular both be brain regions.

Particularly preferably, the neuroactive compound is a neuropsychoactive compound, the neuronal region is a brain region and all neurotransmitter(s) that are object of the method according to the present invention are selected from the group consisting of glutamate, gamma-aminobutyrate, acetylcholine, dopamine, norepinephrine and serotonin.

The method according to the present invention may be conducted for providing a set of clinical data on a healthy subject or a subject suffering from or being at risk of a neuropathologic condition.

In a preferred embodiment, the set of neuronal regions is present in a subject suffering from or being at risk of a neuropathologic condition.

Such neuropathologic condition may be any neuropathologic condition known in the art, i.e., any condition beyond healthy condition. Preferably, the neuropathologic condition is associated with at least one aberrant extracellular local concentration of at least one neurotransmitter and/or with at least one aberrant neuronal network connectivity. Herein, an aberrant extracellular local concentration may be an extracellular local concentration of a neurotransmitter that that deviates by at least +/-10%, more preferably by at least +/-20%, even more preferably by at least +/-30%, even more preferably by at least +/-50%, even more preferably by at least +/-75%, even more preferably by at least -75% to +100%, in particular by at least -75% to +150% from the respective concentration at healthy condition, in particular wherein said extracellular local concentrations are basal extracellular local concentrations. Herein, an aberrant neuronal network connectivity may be understood as a deviation in an effect caused by a stimulus compared to the effect caused by a comparable stimulus in a healthy neurocircuitry. The effect may exemplarily be an alteration in the concentration of another neurotransmitter or electrophysiological properties of neuronal network connectivity or behaviour of the subject of interest.

A neuropathologic condition may preferably be a neurological disease. Exemplarily, such neurological disease may be pathologic post-stroke conditions (e.g., post-stroke depression), Parkinson's disease, Alzheimer's disease, Huntington disease, ischemic stroke, epilepsy, non-epileptic seizure disorder, meningitis, multiple sclerosis, amyotrophic lateral sclerosis, muscular atrophy, ataxia (e.g., amyotaxia), arterial obstructive disease, phakomatosis syndrome, polyneuropathy, muscular dystrophy, myotonia, amyosthenia, migraine, attention deficit hyperactivity disorder (ADHD), addiction and/or craving in the broadest sense (e.g., psychostimulant-addiction, alcohol-addiction, nicotine-addiction, opioid-addiction, cocaine-addiction, tetrahydrocannabinol- (THC)-addiction, etc.), post traumatic stress disorder, a mood disorder in the broadest sense (e.g., depression such as, e.g., major depressive disorder (MDD), bipolar disorder (BD), etc.), a mania (e.g., persecution mania), pathologic obesity, a fear disorder, a stress disorder and/or an anxiety disorder. Where applicable such diseases may optionally be understood in the broadest sense as classified by the International Statistical Classification of Diseases version 10 (ICD-10) in the version effective at the filing date of the present application.

These diseases may optionally be accompanied by one or more clinical symptom(s) such as, e.g., depression, dementia, tremor, mood dysfunctions and/or cognitive dysfunctions. Optionally, a neurological disease may also be manifested psychiatrically as a psychiatric mental disease. A neuropathologic condition may be an acute or a chronic neuropathologic condition. A neuropathologic condition may arise due to any genetic and/or environmental influence. Exemplarily, a neuropathologic condition may originate from an inherent lack of neurotransmitter, may originate from an impairment of a neuronal region (e.g., by means of surgery, accident, injury or tumor), may originate from acute or chronic administration of compounds (e.g., medication or drug, nicotine or ethanol abuse), may originate from ischemia (e.g., due to stroke, pathologic calcification or other plaque formations in the vascular system or an aneurism), mechanical pressure to a nervous strand (e.g., herniated vertebral disk), inflammation (e.g., meningitis) and/or hemorrhage in the neuronal system (e.g., cerebral hemorrhage).

Optionally, the subject (independent whether healthy or suffering from or being at risk of a neuropathologic condition) may be subjected to one or more stimuli provoking feelings such as, e.g., pleasure, pain, fear, stress and/or anxiety in said subject. Further, the subject may optionally also be hypnotized and/or anesthetized.

The provision of a set of clinical data on extracellular local concentrations of one or more neurotransmitter(s) in the absence or presence of one or more neuroactive compound(s) in a set of neuronal regions in a subject suffering from or being at risk of a neuropathologic condition may provide valuable information about the disease/health status of such individual subject and may enable the simulation of alterations upon administration of various amounts of one or more neuroactive compound(s) to the subject. This may also enable to optimize the amount of the one or more neuroactive compound(s) to be administered to the individual subject and may enable the use of said method in personalized medicine.

Moreover, a neurotransmitter may influence the extracellular local concentration(s) of other neurotransmitter(s) either directly (e.g., by triggering the excretion and/or exocytosis of the other neurotransmitter(s)) or indirectly (e.g., by changing the activity potential of a nervous strand leading to the transmission of an electrical current flow and finally the excretion and/or exocytosis of the other neurotransmitter(s)). Preferably, the neurochemical connectivity of interest and, therefore, the extracellular space(s) of interest is/are located in the central nervous system (CNS) including the brain, in particular in a brain region. Alternatively or additionally, the neuronal region(s) may also be muscle(s), in particular one or more neuromuscular junction(s) and/or one or more synapse(s) of the peripheral nervous system.

Furthermore, the neuronal region(s) may also be on a cellular (i.e., a microscopical) level. Then, a neuronal region may exemplarily be the extracellular space of a single synaptic cleft or a connectivity of two or more synaptic clefts. Such synaptic cleft(s) may then, exemplarily, be located in the brain, the CNS, the peripheral nervous system and/or may be neuromuscular junction(s). It has been found that, in particular in the brain, different health states bear distinguishable patterns of interactions between a variety of neurotransmitters, thereby distinguishable extracellular local concentrations of variety of neurotransmitters in different brain regions and particular neurocircuitries (cf., exemplarily, Noori et al. (2012), Addict Biol 17(5):827-864).

Summarized, as laid out above, the method according to the present invention may provide the four-dimensional trajectories (local neurotransmitter concentration x time), which suggest the time course of the neurotransmitter concentrations under basal and neuroactive compound-induced conditions.

Exemplarily, this may be expressed as a time course of the behavior of extracellular local concentration of a neurotransmitter in each neuronal region. Optionally, these results may be normalized by means of dividing the aforementioned concentrations through the basal extracellular local concentration of said neurotransmitter in said neuronal region(s), providing the percental alterations enabling easier comparisons of various results (e.g., expanded data sets and experimentally measured *in vivo* with another.

The method according to the present invention may be performed by using a large variety of algorithms such as, exemplarily, any of the currently available algorithms utilizing higher order Runge-Kutta-method with Hermite or $C^1$ interpolation. Exemplarily, MATLAB (e.g., equipped with the dde23 solver) may be employed.

The extracellular local concentration of a neurotransmitter in a neuronal region as determined by means of the method according to the present invention may be optionally rescaled to reflect individual variabilities of the subject of interest. Since the initial functions as used herein are preferably based on meta-analyses (i.e., the average values of baseline concentrations).

The extracellular local concentration of a neurotransmitter in a neuronal region as determined by means of the method according to the present invention may be optionally rescaled by means of the steps of:

(1) providing clinical data on the basal extracellular local concentrations of the neurotransmitter in at least two neuronal regions *in vivo* in the subject of interest;
(2) rescaling the basal extracellular local concentrations of the neurotransmitter to the concentrations to the concentrations according to said clinical data enabling providing the predicted qualitative behavior; and
(3) performing numerical determination to predict the quantitative behavior of the neurochemical trajectories for said subject of interest.

Herein, the step (1) of providing clinical data may be experimentally determining the basal extracellular local concentrations of the neurotransmitter in at least two neuronal regions *in vivo* in the subject of interest or may be obtained from clinical data restored in a data base.

The method according to the present invention may therefore also be combined with *in vivo* methods such as those based on proteomics, magnetic resonance tomography (MRT), pharmacological MRT based on local cerebral blood perfusion, positron emission tomography (PET), single-photon emission computed tomography (SPECT), microdialysis, voltammetry and/or other biosensors. Then, the results of the method of the present invention may be used to indicate which experimental settings are preferably to be used to obtain suitable results. Exemplarily suitable concentration ranges of one or more neuroactive compound(s) for influencing neurocircuitries may be determined by the present invention. Likewise, the concentration of one or more neuroactive compound(s) to be administered to a subject for diagnostic and/or therapeutic purposes may be determined by the present invention.

As already indicated above, the method according to the present invention may also be used for screening of compounds (in particular of pharmaceutical compounds) in order to provide new and/or improved neuroactive compounds, combinations of neuroactive compounds, dosage regimes thereof and/or administration routes thereof.

Therefore, in a preferred embodiment, the method is a screening method wherein at least one local and/or systemic effect of a compound or a combination of compounds selected from a compound library is determined.

More preferably, the method is a screening method wherein at least one local and/or systemic effect achieved by each of a number of compounds or combinations of compounds selected from a compound library is determined.

Herein, the term "screening method" may be understood in the broadest sense as a systematic test assay usable to determine the local and/or systemic effects of a number of compounds either each alone and/or in combination with another in order to identify compounds or combinations thereof, dosage regimes thereof and/or administration routes thereof that bear particular properties with respect to alterations in neurocircuitries. Exemplarily, each of the compounds of the compound library may be tested one after the other in the method according to the present invention.

A local effect as used herein may be an alteration of one or more extracellular local concentration(s) of one or more neurotransmitter(s) and/or few neuronal interactions in one or few neuronal region(s). A systemic effect as used herein may be an alteration in neurocircuitries in the neuronal regions in a subject's body and/or an alteration in subject's behaviour.

A compound library may be a selection of compounds that are tested as potential neuroactive compounds according to the present invention. The compound library as used herein may comprise a variety of known drugs (know for the intended therapeutic application or for another application), drug candidates, unknown agents and/or combinations of two or more compounds. Further, additionally or alternatively such compounds or combinations thereof may be tested at various dosage regimes. Still further, additionally or alternatively such compounds or combinations thereof may be tested for different administration routes. Therefore, a compound as used in the context of the screening method may be any compound that may potentially have an effect on the extracellular local concentrations and/or neurocircuitries of interest, i.e. may potentially be a neuroactive compound. It may bear the chemical and physical properties as indicated in the context of neuroactive compounds herein.

Exemplarily, the compound may be such already used as a neuroactive drug in the same pharmacologic field as being of interest by the present screening method, therefore, already used for essentially the same therapeutic application, or may be a drug candidate in the same pharmacologic field. For this purpose, preferably, the screening may be used to determine new and/or improved combinations with one or more other neuroactive compound(s), dosage regimes of the neuroactive compound(s) and/or administration routes for the neuroactive compound(s). For this purpose, typically, a reduced set of clinical data for each of a number of compounds is provided experimentally.

Alternatively, the compound may be known as a drug in a different pharmacologic field as being of interest by the present screening method, therefore, so far used for another therapeutic application wherein said compound may be a neuroactive compound or may be a compound used for a completely different pharmaceutical purpose beyond neurocircuitries. Then, it may be tested whether said compound may also be used for the pharmaceutical purpose of interest by the present screening method. The application of a known compound to a new therapeutic use may be also understood as compound repurposing. Herein, the terms "repurposing", "repositioning", "re-profiling", "therapeutic switching" and "re-tasking" may be understood interchangeably. In addition, the screening may be also used to determine new and/or improved combinations of said repurposed compound with one or more other neuroactive compound(s), dosage regimes of the repurposed compound and, optionally, the other neuroactive compound(s), and/or administration routes of the repurposed compound and, optionally, the other neuroactive compound(s). For this purpose, typically, a reduced set of clinical data for each of a number of compounds is provided experimentally.

Exemplarily, the compound library is a selection of compounds that may potentially have an antipsychotic effect by acting as dopamine antagonists at the corpus striatum, the nucleus accumbens and the caudate putamen. Therefore, in this case, the method may be understood as a screening method wherein the dopamine antagonistic effect in the corpus striatum, the nucleus accumbens and the caudate putamen of a compound selected from a compound library comprising candidates for antipsychotic neuroactive compound is determined. Exemplarily, it may be determined that Haloperidol, as far as comprised in the compound library, may serve as an effective neuroactive compound in this context.

As indicated above, the method according to the present invention may be also used to identify new lead structures. For this purpose, typically, a number of potential lead structures are synthesized and a reduced set of clinical data are provided for each of said lead structures experimentally. Likewise, the method may also be used to improve lead structures. For this purpose, typically, a number of variants and derivatives of a lead structure are synthesized and a reduced set of clinical data are provided for each of said lead structures experimentally.

Further, as also indicated above, the method according to the present invention may also be used to determine improved combinations of two or more neuroactive compounds. For this purpose, typically, a reduced set of clinical data for each of a number of combinations of different neuroactive compounds and/or of two or more particular neuroactive compounds is provided experimentally. Likewise, the method according to the present invention may be used to determine improved improve dosage regimes of a neuroactive compound or a combination of neuroactive compounds. For this purpose, typically, a reduced set of clinical data for each of a number of different doses of a neuroactive compound or a combination of neuroactive compounds is provided experimentally. Further, as also indicated above, the method according to the present invention may be used to determine improved administration routes for a neuroactive compound or a combination of neuroactive compounds. For this purpose, typically, a reduced set of clinical data for each of a number of administration routes for a neuroactive compound or a combination of neuroactive compounds is provided experimentally.

Subsequently, any of the aforementioned reduced sets of clinical data provided experimentally, may be expanded by means of the computer-implemented method according to the present invention. This expansion may then provide information on the respective local and/or systemic effect.

Optionally, in such screening method, a threshold with respect to such local and/or systemic effect may be set to distinguish between desired and undesired effects and to filter the hits, i.e., the desired lead structures, combinations, dosages and/or administration routes.

Optionally, in such screening method, two thresholds indicating (i) the minimal therapeutically effective dose of a compound or combination of compounds and (ii) the minimal toxic dose of a compound or combination of compounds may be set to determine an optimized dosage regime of a compound or combination of compounds in the therapeutic but not yet toxic range. The values for such thresholds may depend on the individual subject and on the severity of the disease and may be set adjusted to the individual case. Exemplarily, when treating a severe mental disorder slight side effects may be acceptable whereas these are typically not considered as acceptable when affecting a slight subclinical discomfort.

As laid out above, additionally or alternatively, the step (iv) of rescaling the simulated evolution of extracellular local concentrations of the one or more neurotransmitters may also be performed based on the clinical data on evolution of blood oxygen and/or glucose levels in the absence and/or presence of neuroactive compounds.

Therefore, the present invention further relates to a computer-implemented method for providing a set of clinical data on extracellular local concentrations of one or more neurotransmitters in the absence or presence of one or more neuroactive compounds in a set of neuronal regions, said method comprising the steps of:

(i) providing clinical data on evolution of extracellular local concentrations of the one or more neurotransmitters in the absence and/or presence of neuroactive compounds in a reduced number of neuronal regions from the set of neuronal regions;

(ii) optionally providing the initial concentrations of the one or more neuroactive compounds in each neuronal region of the set of neuronal regions;

(iii) simulating an evolution of extracellular local concentrations of one or more neurotransmitters within a neurocircuitry modelling the extracellular local concentrations of said one or more neurotransmitters in the absence or optionally in the presence of neuroactive compounds and the effect of said initial concentrations of said one or more neuroactive compounds,

wherein the basal extracellular local concentrations of said one or more neurotransmitters are averaged basal extracellular local concentrations; and

(iv) rescaling the simulated evolution of extracellular local concentrations of the one or more neurotransmitters by the clinical data on evolution of blood oxygen and/or glucose levels in the absence and/or presence of neuroactive compounds in a reduced number of neuronal regions,

whereby the clinical data on evolution of blood oxygen and/or glucose levels in the absence and/or presence of neuroactive compounds in the reduced number of neuronal regions is extended to clinical data on extracellular local concentrations of the one or more neurotransmitters in the absence or presence of one or more neuroactive compounds for each neuronal region of the set of neuronal regions.

This method is preferably also characterized as laid out above, in particular by one of the preferred embodiments.

Moreover, the present invention relates to a computer-implemented method for providing a set of clinical data on blood oxygen and/or glucose levels in the absence or presence of one or more neuroactive compounds in a set of neuronal regions, said method comprising the steps of:

(i) providing clinical data on evolution of extracellular local concentrations of the one or more neurotransmitters in the absence and/or presence of neuroactive compounds in a reduced number of neuronal regions from the set of neuronal regions;

(ii) optionally providing the initial concentrations of the one or more neuroactive compounds in each neuronal region of the set of neuronal regions;

(iii) simulating an evolution of extracellular local concentrations of one or more neurotransmitters within a neurocircuitry modelling the extracellular local concentrations of said one or more neurotransmitters in the absence or optionally in the presence of neuroactive compounds and the effect of said initial concentrations of said one or more neuroactive compounds,

wherein the basal extracellular local concentrations of said one or more neurotransmitters are averaged basal extracellular local concentrations; and

(iv) rescaling the simulated evolution of extracellular local concentrations of the one or more neurotransmitters by the clinical data on evolution of blood oxygen and/or glucose levels in the absence and/or presence of neuroactive compounds in a reduced number of neuronal regions,

whereby the clinical data on evolution of blood oxygen and/or glucose levels in the absence and/or presence of neuroactive compounds in the reduced number of neuronal regions is extended to clinical data on extracellular local concentrations of the one or more neurotransmitters in the absence or presence of one or more neuroactive compounds for each neuronal region of the set of neuronal regions.

This method is preferably also characterized as laid out above, in particular by one of the preferred embodiments.

As laid out above, the method may also enable to optimize the amount of the one or more neuroactive compound(s) to be administered to the individual subject suffering from or being at risk of a neuropathologic condition and may enable the use of said method in personalized medicine.

Accordingly, a further aspect of the present invention relates to a composition comprising one or more neuroactive compounds for use in a method for treating or preventing a subject suffering from or being at risk of a neuropathologic condition, wherein said subject is administered with an amount of said composition sufficient to restore the neurocircuitry associated with said neuropathologic condition to a non-pathologic state, said amount determined by:

(i) providing clinical data on evolution of extracellular local concentrations of one or more neurotransmitters associated with said neuropathologic condition in the absence and/or presence of neuroactive compounds in a reduced number of neuronal regions from the set of neuronal regions associated with said neuropathologic condition in said subject according to the method according to the present invention;

(ii) simulating an evolution of extracellular local concentrations of the one or more neurotransmitters within a neurocircuitry modelling the extracellular local concentrations of said one or more neurotransmitters in the presence of various amounts of said composition and the effect of the resulting initial concentrations of said one or more neuroactive compounds according to the method according to the present invention; and

(iii) selecting the amount of said composition sufficient to restore the neurocircuitries to a non-pathologic state.

It will be understood that the definitions and explanations of the method and terms made above apply *mutatis mutandis* for the composition for use.

A composition comprising one or more neuroactive compound(s) may optionally further comprise a pharmaceutically acceptable carrier.

As used herein, the term "pharmaceutically acceptable carrier" may refer to any substance that may support the pharmacological acceptance of the inhibitor. The pharmaceutical composition may be administered orally or may be injected. It may be pharmaceutically formulated in a dry form (e.g., as a powder, a tablet, a pill, a capsule, a chewable capsule, etc.) or a liquid (e.g., a spray, a syrup, a juice, a gel, a liquid, a paste, an injection solution, an aerosol, an enema, etc.) A pharmaceutically acceptable carrier may be a solvent with no or low toxicity such as, e.g., an aqueous buffer, saline, water, dimethyl sulfoxide (DMSO), ethanol, vegetable oil, paraffin oil or combinations thereof. Furthermore, the pharmaceutically acceptable carrier may contain one or more detergent(s), one or more foaming agent(s) (e.g., sodium lauryl sulfate (SLS), sodium doceyl sulfate (SDS)), one or more coloring agent(s) (e.g., $TiO_2$, food coloring), one or more vitamin(s), one or more salt(s) (e.g., sodium, potassium, calcium, zinc salts), one or more humectant(s) (e.g., sorbitol, glycerol, mannitol, propylenglycol, polydextrose), one or more enzyme(s), one or more preserving agent(s) (e.g., benzoic acid, methylparabene), one or more texturing agent(s) (e.g., carboxymethyl cellulose (CMC), polyethylene glycol (PEG), sorbitol), one or more emulsifier(s), one or more bulking agent(s), one or more glacing agent(s), one or more separating agent(s), one or more antioxidant(s), one or more herbal and plant extract(s), one or more stabilizing agent(s), one or more polymer(s) (e.g., hydroxypropyl methacrylamide (HPMA), polyethylene imine (PEI), carboxymethyl cellulose (CMC), polyethylene glycol (PEG)), one or more uptake mediator(s) (e.g., polyethylene imine (PEI), dimethyl sulfoxide (DMSO), a cell-penetrating peptide (CPP), a protein transduction domain (PTD), an antimicrobial peptide, etc.) one or more antibody/antibodies, one or more sweetener(s) (e.g., sucrose, acesulfam K, saccharin Na, stevia), one or more counterstain dye(s) (e.g., fluorescein, fluorescein derivatives, Cy dyes, an Alexa Fluor dyes, S dyes, rhodamine, quantum dots, etc.), one or more gustatory substance(s) and/or one or more fragrance(s).

Examples for neuroactive compounds and neuropathologic conditions are provided above. The person skilled in the art will know which composition comprising one or more neuroactive compound(s) is generally suitable for treating or preventing which neuropathologic condition. Additionally, the person skilled in the art will know the respective therapeutic ranges of each neuroactive compound suitable to avoid adverse effects as far as possible. The effect of a neuroactive compound may be a direct effect on the respective neurotransmitter concentration(s) and/or may alter one or more other neurotransmitter concentration(s) dire indirectly altering the neurotransmitter concentration(s) of interest.

The step (i) of providing clinical data on evolution of extracellular local concentrations of one or more neurotransmitters may be understood in the broadest sense as laid out above. Therefore, the extracellular local concentration may be determined from data obtainable from a data base or may be measured or have been measured *in vivo* in the patient (e.g., by means of microdialysis (in particular dual-probe microdialysis), voltammetry, proteomics, magnetic resonance tomography (MRT), pharmacological MRT based on local cerebral blood perfusion, positron emission tomography (PET), single-photon emission computed tomography (SPECT) and/or other biosensors).

Preferably, the extracellular local concentration of a set of neurotransmitters may be determined by measuring said concentration *in vivo* in the subject by means of an invasive method (e.g., microdialysis, voltammetry) non-invasive method (e.g., by means of magnetic resonance tomography (MRT), pharmacological MRT based on local cerebral blood perfusion, positron emission tomography (PET), single-photon emission computed tomography (SPECT) and/or other biosensors). This measuring step may or may not form a step of the present method.

The step (ii) of simulating an evolution of extracellular local concentrations of the one or more neurotransmitters within a neurocircuitry associated with said neuropathologic condition may also include metabolic factors of the patient as laid out in the context of neuroactive compounds above. Therefore, for determining the amount of a neuroactive composition to be administered the body weight, the body fat percentage, the body surface, the route of administration and/or the individual metabolic characteristics of the subject (e.g. whether the subject is a slow, medium, rapid or ultra-rapid metabolizer) may play a role. The concentration of the neuroactive compound will typically alter over time (e.g., essentially following a Bateman function).

The step (iii) of selecting the amount of said composition sufficient to restore the neurocircuitries to a non-pathologic state may exemplarily be selecting the amount of said neuroactive compound that is sufficient to restore the neurocircuitries to a non-pathologic state at least for a certain time interval. Preferably, the amount administered is below the dose causing intoxication and above the therapeutic threshold for essentially the entire time determined.

Administration may be administration once (e.g., as a bolus administration) or may be administration according to an administration scheme preferably maintaining the body concentration of the administered composition below the dose causing intoxication and above the therapeutic threshold for essentially the entire time determined. The composition may also be a retard composition.

Exemplarily, when the patient suffers from Parkinson's disease, the present invention may relate to a composition comprising one or more dopamine agonist(s) for use in a method for treating a subject suffering from Parkinson's disease, wherein said subject is administered with an amount of the composition sufficient to restore the neurocircuitry associated with Parkinson's disease to a non-pathologic state, said amount determined by:

(i) providing the extracellular local dopamine concentration in at least the *substantia nigra* in said subject;
(ii) simulating the effect of a set of amounts of said composition on the neurocircuitries associated with Parkinson's disease of said subject by means of the method according to the present invention; and
(iii) selecting the amount of said composition sufficient to restore the neurocircuitries to a non-pathologic state.

As used herein, dopamine agonist may be any direct or indirect dopamine agonist that is able to pass the blood-brain barrier or which precursor is able to pass the blood-brain barrier. Exemplarily, a dopamine agonist may be selected from the group consisting of DOPA, bromocriptine, pergolide, pramipexole, ropinirole, piribedil, cabergoline, Apomorphine, rotigotine, lisuride, amphetamins, catechol-O-decarboxylase inhibitor (COMT) inhibitors and/or monoamine oxidase type B (MAO-B) inhibitors (e.g., selegiline and/or rasagiline).

Exemplarily, a neuroactive compound may also be an antpsychotic neuroactive compound (e.g., haloperidol) and the subject may be a psychotic patient bearing increased basal extracellular local concentration of the neurotransmitter dopamine in the brain regions corpus striatum, nucleus accumbens and caudate putamen.

Exemplarily, a neuroactive compound may also be such acting indirectly may be a selective serotonin reuptake inhibitor (SSRI) neuroactive compound (e.g., fluoxetine) and the subject may bear an increased basal extracellular local concentration of the neurotransmitter dopamine in the brain regions insular cortex.

Exemplarily, the composition comprising one or more neuroactive compound(s) may also comprise a acetylcholine esterase inhibitor (e.g., tacrine, rivastigmine, galantamine or donepezil) and/or an NMDA receptor antagonist and the subject may be suffering from Alzheimer's disease bearing a decreased basal extracellular local concentration of the neurotransmitter acetylcholine in at least one brain region and/or an increased basal extracellular local concentration of the neurotransmitter glutamate in at least one brain region.

Exemplarily, the composition comprising one or more neuroactive compound(s) may also comprise a dopamine and/or norepinephrine reuptake inhibitor (e.g., methylphenidate) and/or an amphetamine (e.g., atomotexine) and the subject may be suffering from attention deficit hyperactivity disorder (ADHD) bearing a decreased basal extracellular local concentration of the neurotransmitter(s) dopamine and/or epinephrine.

Exemplarily, the composition comprising one or more neuroactive compound(s) may also comprise an antiepilepticum (e.g., valproinic acid) and the subject may be suffering from or being at risk of epilepsy and bears an increased extracellular local concentration of glutamate in at least one brain region, an increased extracellular local concentration of aspartate in at least one brain region and/or a decreased extracellular local concentration of gamma-amino butyrate in at least one brain region. Optionally, the subject also bears extracellular local concentrations of one or more neuropeptide(s) (e.g., neuropeptide Y and/or galanine) are decreased in at least one brain region.

The amount of the composition comprising certain amounts of one or more neuroactive compound(s) can be determined unambiguously clear by means of the method according to the present invention. Therefore, when particular parameters are used in the method according to the present invention (i.e., particular neurocircuitries based on particular extracellular local concentrations of neurotransmitter(s) in particular neuronal region(s) in a particular patient), then the amount of the composition comprising one or more neuroactive compound(s) can be unambiguously determined to restore the neurocircuitry associated with said neuropathologic condition to a non-pathologic state.

The determination of the required amount may be carried out for each subject to be administered with a composition individually (personalized medicine) or may be determined for a particular population of subjects such as, e.g., subjects bearing a particular health status, subjects at a certain age range and/or of certain sex.

A yet further aspect of the present invention relates to a method for treating or preventing a subject suffering from or being at risk of a neuropathologic condition, wherein said subject is administered with an amount of a composition comprising one or more neuroactive compound(s) sufficient to restore the neurocircuitry associated with said neuropathologic condition to a non-pathologic state, said amount determined the above steps.

As laid out above, the computer-implemented method according to the present invention may be conducted on any kind of computer, i.e., any device that is programmable to carry out a set of arithmetic and/or logical operations.

Accordingly, a further aspect of the present invention relates to a computer program product directly loadable into the internal memory of a digital computer, comprising software code portions suitable for implementing the provision of a set of clinical data on extracellular local concentrations of one or more neurotransmitter(s) in the absence or presence of one or more neuroactive compound(s) in a set of neuronal regions according to the method of the present invention when said product is run on a computer.

It will be understood that the definitions and explanations of the method and terms made above apply *mutatis mutandis* for the computer program product.

According to the invention, a computer program product is provided for performing the method according to the present invention, when the product is run on a computer. The computer program product is preferred to be directly loadable into the internal memory of a digital computer and comprises software code portions for providing a set of clinical data on extracellular local concentrations of one or more neurotransmitter(s) in the absence or presence of one or more neuroactive compound(s) in a set of neuronal regions.

The computer program product may be a computer program preferably stored on a machine readable storage medium like RAM, ROM, or on a removable CD-ROM, flash memory, DVD or USB-stick. The computer program may be provided on a server to be downloaded via for example a data network such as the internet or another transfer system such as a phone line or a wireless transfer connection. Additionally, or alternatively, the computer program product may be a network of computer implemented computer programs such as a client/server system or a cloud computing system, an embedded system with a computer program or an electronic device like a smart phone or a personal computer on which a computer program is stored, loaded, running, exercised, or developed.

In one implementation, the information on clinical data on evolution of extracellular local concentrations of the one or more neurotransmitter(s) in the absence of neuroactive compounds in a reduced number of neuronal regions from the set of neuronal regions is entered into an input device of a computer, an embedded system, an electronic device or a smart phone via a keyboard, a touch screen or a voice interface. In another implementation, the information on the clinical data is measured by adequate measuring devices and transmitted via the internet or another transfer system such as a phone line or a wireless transfer connection to a remote station such as a computer.

In one implementation, the information on the evolution of extracellular local concentration(s) of one or more neurotransmitter(s) within a neurocircuitry is provided on an output medium, stored in a memory means or transferred to a remote station. The information on the evolution of extracellular local concentration(s) of one or more neurotransmitter(s) within a neurocircuitry may also be displayed on a screen or display as output medium.

The following figures and examples are intended to illustrate the invention but not to limit the scope of protection conferred by the claims.

Figures

Figure 1 shows the workflow of the method containing six interacting computational and experimental schemes.

Figure 2 shows the neurocircuitry for modeling neuroactive compound effects (lowest bar) encompasses the neurocircuitries for mood disorders, anxiety disorders and addiction and thereby provides an appropriate framework to investigate co-morbidities, interactions of different pharmacologically active neuroactive compounds and ultimately neuroactive compound-repurposing.

Figure 3 demonstrates an enhancement in the basal dopamine levels in nucleus accumbens of around 150% upon chronic alcohol administration as confirmed by Sommer and colleagues experimentally.

Figure 4 shows the numerical simulations (white bars) of the effects of the acute intraperitoneal administration of A) 1g/kg ethanol and B) 10 mg/kg fluoxetine on the monoamine concentrations show no significant differences with post-simulation *in vivo* microdialysis measurements (black bars) of the neurotransmitters in nucleus accumbens and insular cortex of rats, respectively.

Examples

1. Design and Workflow of the Method

The applied method generally bases on assumption-free constructions of neurocircuitries (Noori et al., 2012a). Two types of data were considered for constructing the neurocircuitry: (1) information on the cytoarchitecture and neurochemical connectivity of each brain region obtained from different neuroanatomical techniques; (2) information on the functional relevance of each neuronal region with respect to alcohol and neuroactive compound effects. To identify the components of the neurocircuitry, a successive data-mining method derived from PubMed articles (http://www.ncbi.nlm.nih.gov/pubmed) (Box 1 of Figure 1) was applied.

All PubMed articles (over 20 million scientific publications) were screened and systematically filtered based on fixed criteria. First, 2.3 million original research articles on rodents were retrieved and then keywords of neuroanatomical tracing techniques and neurochemical localization methods to screen the literature for studies regarding the topology of different brain regions have been applied. For this, information on the cytoarchitecture and neurochemical connectivity of each brain region obtained using different neuroanatomical techniques, including the anterograde degeneration, anterograde tracing with radioactive amino acids, and autoradiographic fiber tracing methods, as well as anterograde and retrograde tracing with horseradish peroxidase was used. This collection of scientific publications of anatomical and neurochemical information was then refined by excluding studies on interneurons. By divisive hierarchical clustering implemented as Girvan-Newman algorithm (using edge betweenness as weights), the maximal number of brain regions with anatomical information on their efferents and afferents was determined. By eliminating studies on neural projections without chemical co-localizations and inconsistencies regarding mapping studies and colocalization studies, an anatomical database of efferent and afferent connections of brain regions with their neurotransmitter identification was obtained (Noori et al. (2012), Addict Biol 17(5):827-864).

This data-mining strategy provides for each neurotransmitter system *Tr* two characteristics: 1) *Rg(Tr),* which represents the number of network compartments that contain the neurotransmitter *Tr* and 2) *Conn(Tr),* which denotes the number of efferents within the clustered network utilizing *Tr.* The ratio of *Conn(Tr)* to *Rg(Tr)* is a measure of the goodness of the data mining process as well as the sufficiency of the available experimental data to capture the functional role of *Tr* within the network. Therefore, this ratio was used to define the final criterion for exclusion of neurotransmitter systems from the present structure of our neurocircuitry.

If for a neurotransmitter Tr the ratio of *Conn(Tr)* to *2xRg(Tr)* is smaller than 0.5 (below equation), and thus the data mining failed to capture less than 50% of the functional connectivity of this neurotransmitter system, then the neurotransmitter Tr was excluded from the present design of the neurocircuitry $0.5 > \dfrac{Conn(Tr)}{2 \times Rg(Tr)}$.

The neurocircuitry for modeling neuroactive compound effects has been demonstrated in detail (Noori et al. (2012), Addict Biol 17(5):827-864, in particular Figure 1, wherein the global connectivity map characterizes the topological structure of the neurocircuitry by three graphically encrypted measures: 1) the radius of the connections qualitatively reflects the anatomical distances along the Bregma levels, 2) the thickness of the connections is a measure for their chemical diversity, i.e. the thicker the line the more neurotransmitters are co-localized along the bilateral projections, and 3) the connectivity pattern of the principal core-subcircuitry (green lines)). An individual representation of the subcircuits of the chemical neuroactive compounds with significant anatomical connectivities has also been demonstrated in detail (Noori et al. (2012), Addict Biol 17(5):827-864, in particular Figure 2, wherein the topological structure of the subcircuits is preserved by the radius of the connections as a measure for the bilateral distances along the Bregma levels).

1.1. The Mathematical Model

Based on the neurocircuitry for modeling neuroactive compound effects, the present model is a system of 88 coupled non-linear delay-differential equations of the form

$$\frac{ds_l^k(t)}{dt} = \sum_{\substack{m=1 \\ m \neq l}}^{\deg^{out}(l)} -f_{lm}^k(t)s_l^k(t) + r_l^k(t-\tau_{lm})s_m^k(t-\tau_{lm}) + \sum_{\substack{j=1 \\ j \neq l}}^{\deg^{in}(l)} f_{jl}^k(t-\tau_{jl})s_j^k(t-\tau_{jl}) - [s_l^k(t-\tau_\alpha) - s_l^k(t-2\tau_\alpha)]$$

describing the dynamics of the extracellular local concentration $s_l^k(t)$ of the neurotransmitter k (glutamate, GABA, acetylcholine, dopamine, norepinephrine, and serotonin) in the brain region *l* within the neurocircuitry for modeling neuroactive compound effects (Noori and Jäger, 2010; Noori, 2012a,b; Noori et al., 2012a). The vector field of each equation is comprised by four functional components, which will be explained in detail in the following:

1.1.1. Release:

$$\sum_{\substack{m=1 \\ m\neq l}}^{\deg^{out}(l)} -f_{lm}^{k}(t)s_{l}^{k}(t)$$

Let $deg^{in}(l)$ be the in-degree i.e. number of incoming projections or afferents to a node $l$ in the network defined by neurocircuitry for modeling neuroactive compound effects. Analogous, $deg^{out}(l)$ represents the out-degree i.e. the number of outgoing projections from region $l$. $deg^{in}(l) = Ex(l) + Inh(l)$ is decomposable into $Ex(l)$ the number of excitatory and $Inh(l)$ the number of inhibitory afferents. Let us now define the functions

$$A_l(t) := \frac{1}{Ex(l)} \sum_{j=1}^{Ex(l)} \omega^k \frac{s_j^k(t) - s_j^k(0)}{s_j^k(0)} \quad \text{and} \quad B_l(t) := \frac{1}{Inh(l)} \sum_{p=1}^{Inh(l)} \omega^q \frac{s_p^q(t) - s_p^q(0)}{s_p^q(0)},$$

with k and q representing the exact type of neurotransmitter utilized for the neuronal projection towards $l$ and $\omega_k$ and $\omega_q$ their estimated weight parameters, respectively. The weight parameters are set as $\omega_k = \omega_q = 1$ for the initial simulations, however these values may change after chronic neuroactive compound exposure and an *a posteriori* parameter estimation is required to obtain their values for neuroadaptive states. The function is called $F_l(t)=A_l(t)-B_l(t)$ with $F_l(0)=0$, the neurochemical balance function at node $l$. The function $F_l(t)$ serves as the variable within the saturation function

$$\Gamma_l(t) \in [0,1] \quad \text{with} \quad \Gamma_l(t) = 1 - \frac{1}{e^{4F(t)}+1},$$

the so-called activity function, which in a similar manner as the hysteresis-like operators acts as a switch for neurotransmitter dynamics. The definition of the activity function suggests that a brain region $l$ is activated if and only if the balance of neurochemical afferents into $l$ is changed significantly in favor of excitatory inputs. If however the inhibitory input changes the neurochemical balance, then the region becomes gradually de-activated. Let $a_{lm}^k > 0$ be a real-valued parameter representing the averaging limit of the amplitudes of the sigmoidal release functions induced by changes in normalized membrane potentials, which is estimated for simulations at minute scale by non-linear parameter estimations to reproduce the initial functions.

$$\sum_{\substack{m=1 \\ m\neq l}}^{\deg^{out}(l)} a_{lm}^{k}$$

therefore, represents the ratio of the maximal released neurotransmitters with respect to the total number of neurotransmitters in the same region. Thus, the non-linear operator $f_{lm}^{k}(t) = a_{lm}^{k}\Gamma_l(t)$ represents the utilization of neurotransmitter k at brain region $l$ for projections towards brain region m.

Thus,

$$\sum_{\substack{m=1 \\ m\neq l}}^{\deg^{out}(l)} -f_{lm}^{k}(t)s_{l}^{k}(t)$$

is considered as the functional term for the release of neurotransmitters (loss of mass) towards target regions within the neurocircuitry and contains network topology and functional neurochemistry in its structure.

1.1.2. Resorption:

$$r_l^k(t\text{-}\tau_{lm})s_m^k(t\text{-}\tau_{lm})$$

Herein, $r_l^k(t-\tau_{lm}) = \mu_l^k \Gamma_l(t - \tau_{lm})$ represents the amount of neurotransmitters that are resorbed by projection neurons at target region m after they have been released. Due to metabolism of neurotransmitters, extra-synaptic diffusion processes and similar events, not every neurotransmitter released is resorbed. This factual loss is included within the parameter $\mu^k_m$. The time-lags $\tau_{lm}$ represent the time that is required for a signal to reach region m from region *l* in order to induce the release of neurotransmitters in the target region.

1.1.3. Afferent Projections:

$$\sum_{\substack{j=1 \\ j \neq l}}^{\deg^{in}(l)} f_{jl}^k\big(t\text{-}\tau_{jl}\big)s_j^k\big(t\text{-}\tau_{jl}\big)$$

This term represents the portion of the released neurotransmitter k from neighboring regions j that reaches brain region *l*.

1.1.4. Regeneration:

$$[s_l^k(t\text{-}\tau_\alpha)\text{-}s_l^k(t\text{-}2\tau_\alpha)]$$

Various mechanisms in the brain are responsible for a continuous maintenance of the brain neurochemistry. Through synthesis of precursors to neurotransmitters or metabolism and decomposition of neurotransmitters into smaller elements, the brain regulates and stabilizes the local concentrations of neurotransmitter systems to preclude their affluence or shortage. This mathematical term models these regulatory mechanisms in a very simple manner. Basically, it represents the deficit in neurotransmitter concentration of type **k** in region **l** after a complete cycle of information processing.

For $\tau_\alpha = max_{l \neq m}\tau_{lm}$, if

$$s_l^k(t\text{-}\tau_\alpha)\text{-}s_l^k(t\text{-}2\tau_\alpha)>0$$

,

then the current concentration is reduced by the deficit. If however,

$$s_l^k(t\text{-}\tau_\alpha)\text{-}s_l^k(t\text{-}2\tau_\alpha)<0$$

,

then the lacking concentration is added to the system to ensure the maintenance of the baseline concentration level in absence of stimuli. The time delays in the vector fields do not only represent the time intervals of information processing pathways moreover they generate intrinsic memories of the systems. In other words they define anatomical memories based on the neurochemical connectomics. One of the benefits of the existence of such natural memories reveals itself in the simulations of chronic neuroactive compound effects as the system shows great sensitivity to the patterns of neuroactive compound administration.

1.2. Initial functions

Since the delay differential equations are infinite dimensional, each trajectory is associated with an initial function. In a series of meta-analysis studies (Noori et al., 2012b; Brand et al., 2013; Fliegel et al., 2013), it has been obtained experimentally valid averages of baseline extracellular local concentrations of the neurotransmitter within the neurocircuitry for modeling neuroactive compound effects. While the average values are considered as the initial values at *t=0* (Table 1), the initial functions for each variable $s_i^k(t)$ are generated as continuous and differentiable interpolation of a series of values $s_i^k(0)+\varepsilon$ within the interval [$\tau\alpha$,0], with $\varepsilon$ representing a very small perturbation within the standard deviation of the average values.

**Table 1.** The average baseline levels of neurotransmitter concentrations $s_i^k(0)$

| Substance | Region | Basal Value (nM) |
| --- | --- | --- |
| Glu | OB | 3856.60 |
| NA | OB | 2.30 |
| 5-HT | OB | 0.20 |
| Glu | PFC | 1181.74 |
| ACh | PFC | 28.89 |
| NA | PFC | 1.05 |
| DA | PFC | 0.48 |
| 5-HT | PFC | 0.82 |
| Glu | Ins | 1200.00 |
| NA | Ins | 1.11 |
| DA | Ins | 2.00 |
| 5-HT | Ins | 0.30 |
| Glu | Acb | 2134.88 |
| GABA | Acb | 89.53 |
| ACh | Acb | 35.55 |
| DA | Acb | 4.41 |
| 5-HT | Acb | 2.19 |
| NA | CPu | 1.17 |
| Glu | CPu | 1008.78 |
| GABA | CPu | 17.15 |
| ACh | CPu | 143.97 |
| DA | CPu | 5.12 |
| Glu | S | 3893.80 |
| GABA | S | 639.71 |
| ACh | S | 12.99 |
| DA | S | 0.87 |
| NA | S | 1.13 |
| Glu | BST | 830.00 |

(continued)

| Substance | Region | Basal Value (nM) |
|---|---|---|
| GABA | BST | 110.00 |
| DA | BST | 0.78 |
| NA | BST | 2.44 |
| 5-HT | BST | 0.32 |
| Glu | GP | 434.63 |
| GABA | GP | 21.33 |
| Glu | HyT | 1177.66 |
| GABA | HyT | 28.61 |
| ACh | HyT | 39.87 |
| NA | HyT | 0.50 |
| 5-HT | HyT | 0.30 |
| Glu | Amy | 4475.26 |
| GABA | Amy | 56.24 |
| ACh | Amy | 30.43 |
| NA | Amy | 0.60 |
| 5-HT | Amy | 0.94 |
| DA | Amy | 0.96 |
| Glu | Hb | 4310.04 |
| GABA | Hb | 183.32 |
| ACh | Hb | 32.11 |
| NA | Hb | 0.50 |
| DA | Hb | 0.32 |
| 5-HT | Hb | 0.34 |
| Glu | Hc | 2615.67 |
| GABA | Hc | 96.60 |
| ACh | Hc | 41.98 |
| NA | Hc | 0.41 |
| DA | Hc | 0.24 |
| 5-HT | Hc | 0.34 |
| Glu | Th | 841.51 |
| GABA | Th | 228.36 |
| ACh | Th | 0.41 |
| NA | Th | 1.39 |
| DA | Th | 0.21 |
| 5-HT | Th | 0.29 |
| Glu | STh | 117.60 |
| GABA | STh | 8.79 |
| DA | STh | 0.09 |

(continued)

| Substance | Region | Basal Value (nM) |
|---|---|---|
| Glu | SN | 135.92 |
| GABA | SN | 17.69 |
| DA | SN | 0.30 |
| ACh | SN | 4.14 |
| 5-HT | SN | 0.30 |
| Glu | VTA | 205.27 |
| GABA | VTA | 16.35 |
| DA | VTA | 0.67 |
| ACh | VTA | 5.65 |
| 5-HT | VTA | 0.29 |
| Glu | Raphe | 1243.00 |
| ACh | Raphe | 3.45 |
| DA | Raphe | 5.76 |
| 5-HT | Raphe | 0.87 |
| Glu | LC | 2430.50 |
| DA | LC | 0.40 |
| NA | LC | 2.41 |
| 5-HT | LC | 0.18 |
| Glu | Pn | 75.00 |
| GABA | Pn | 90.00 |
| NA | Pn | 15.07 |
| ACh | Pn | 14.67 |

2. Parameters

2.1. Time Delays

The neurocircuitry for modeling neuroactive compound effects is constituted by 340 neuronal projections connecting 19 brain regions. Due to the symmetry property of the time delays, $\tau_{lm} = \tau_{ml}$, numerical simulation of the mathematical model relies on the knowledge of 340 time delays. While a few electrophysiological studies provide experimentally obtained values for the time delays, the vast majority of these parameters are yet unknown. Therefore, a mathematical method was applied to estimate the time delays based on the already measured values. The method assumes a physically plausible linear relationship between the anatomical distances of projections and time delays (x=vτ). In order to apply this method efficiently, the brain regions of the neurocicruitry were clustered based on their anatomical location within the rodent brain (using Bregma coordinates from rostral to caudal) into six distinct groups: Cluster I (OB), cluster II (PFC, Ins, Acb, CPu, S), cluster III (BST, GP, HyT, Amy), cluster IV (Hb, Hc, Th, STh), cluster V (SN, VTA), and cluster VI (R, LC, Pons). Using the previously measured propagation times along neuronal projections in the rodent brain, an average propagation speed of 1.985 m/s as the correlating factor of distances and time delays and estimated subsequently the time delays for the identified clusters has been calculated (Table 2).

**Table 2.** Estimated projection time delays.

| Time-lags $\tau_{lm}$ [ms] | Cluster II | Cluster III | Cluster IV | Cluster V | Cluster VI |
|---|---|---|---|---|---|
| Cluster I | 2.6 | 4 | 5.1 | 6.2 | 7.8 |

(continued)

| Time-lags $\tau_{lm}$ [ms] | Cluster II | Cluster III | Cluster IV | Cluster V | Cluster VI |
|---|---|---|---|---|---|
| Cluster II | | 2 | 2.5 | 3.6 | 5.3 |
| Cluster III | | | 1.1 | 2.2 | 3.8 |
| Cluster IV | | | | 1.1 | 2.7 |
| Cluster V | | | | | 1.7 |

Numerical simulation of delay differential equations is computionally very expensive and its complexity increases with the number of time delays that constitute the memory of the system. Therefore, the time delays are rounded up to integers for simulations to reduce the complexity of the system and enhance its efficacy (Table 3).

**Table 3.** The time delays used for numerical simulations.

| Time-lags $\tau_{lm}$ [ms] | Cluster II | Cluster III | Cluster IV | Cluster V | Cluster VI |
|---|---|---|---|---|---|
| Cluster I | 3 | 4 | 5 | 6 | 8 |
| Cluster II | | 2 | 3 | 4 | 5 |
| Cluster III | | | 1 | 2 | 4 |
| Cluster IV | | | | 1 | 3 |
| Cluster V | | | | | 2 |

2.2. System Parameters $a_{lm}{}^k$ and $\mu_m{}^k$

The system parameters $0 < \mu_m{}^k a_{lm}{}^k < 1$, are estimated in the interval $[\tau_\alpha, 0]$ using the initial functions with $\Gamma_l(t)$ = **1** as the reference experimental values and the sum

$$\sum_{\substack{m=1 \\ m \neq l}}^{\deg^{\mathbf{out}}(l)} a_{lm}^k = 1$$

representing the ratio of maximal utilized quantity for neuronal interactions and total extracellular local concentration of the neurotransmitter, as constraints. In order to overcome the under-determinedness of the system with respect to the system parameters, it was assumed that $\forall m, \mu_{mm}^k = \mu^k$. In other words, the metabolism parameters of the system are proposed to differ with respect to the chemical structure of the neurotransmitters but to not differ significantly among different brain regions. Based on this assumption, the estimated $\mu^k$ parameters (Table 4) suggest that the neurochemical systems in active state resorb 90% to 97% of the released neurotransmitters.

**Table 4.** Estimated metabolism parameters $\mu^k$ vary between 3-10%.

| $\mu^{glu}$ | $\mu^{GABA}$ | $\mu^{Ach}$ | $\mu^{NA}$ | $\mu^{DA}$ | $\mu^{5\text{-}HT}$ |
|---|---|---|---|---|---|
| 0.101917001 | 0.061747974 | 0.076373644 | 0.050773438 | 0.03053783 | 0.039261928 |

To improve the computational properties of the system, numerical simulations are best conducted on the non-dimensionalized system. For this purpose, the parameters of the non-dimensionalized system are estimated (Table 5). It should be remarked that the non-dimensionalized parameters do not fulfill the condition: $0 < \mu_m{}^k$, $a_{lm}{}^k < 1$

**Table 5.** Estimated non-dimensionalized metabolism parameters $\mu^k$.

| $\mu^{glu}$ | $\mu^{GABA}$ | $\mu^{Ach}$ | $\mu^{NA}$ | $\mu^{DA}$ | $\mu^{5\text{-}HT}$ |
|---|---|---|---|---|---|
| 1.560562666 | 1.296405421 | 0.074788416 | 0.496674341 | 0.02233399 | 0.064278554 |

Under the same conditions, the simulation parameters $a_{lm}{}^k$ were estimated (Table 6 and 7 for non-dimensionalized system). The indices $l, m \in \{1,...,6\}$ and $k \in \{1,...,6\}$ represent the brain regions and neurotransmitters.

Indices $l$ and $m$ denote 1: Olfactory bulb; 2: Prefrontal cortex; 3: Insula; 4: Nucl. accumbens; 5: Caudateputamen; 6: Septal region; 7: Bed nucl. of stria terminalis; 8: Globus pallidus; 9: Hypothalamus; 10: Amygdala; 11: Habenula; 12: Hippocampus; 13: Thalamus; 14: Subthalamic nucl.; 15: Substantia nigra; 16: Ventral tegmental area; 17: Raphe nucl.; 18: Locus coeruleus; 19: Pons.

Index $k$ denotes 1: Glutamate; 2: GABA; 3: Acetylcholine; 4: Norepinephrine; 5: Dopamine and 6: Serotonin.

**Table 6.** Estimated system parameters $a_{lm}{}^k$ (l,m,k = Origin, Target, Neurotransmitter).

| l,m,k | $a_{lm}{}^k$ | l,m,k | $a_{lm}{}^k$ | l,m,k | $a_{lm}{}^k$ |
|---|---|---|---|---|---|
| 1,3,1 | 0.077708309 | 9,19,2 | 2.306368836 | 15,4,5 | 0.00001 |
| 1,9,1 | 0.180223927 | 9,2,3 | 0.779945688 | 15,5,5 | 2.213936534 |
| 1,13,1 | 0.00001 | 9,7,4 | 0.00001 | 15,6,5 | 0.00001 |
| 2,3,1 | 0.00001 | 9,10,4 | 0.00001 | 15,7,5 | 0.00001 |
| 2,4,1 | 0.00001 | 9,11,4 | 0.00001 | 15,10,5 | 0.00001 |
| 2,5,1 | 0.307972518 | 9,12,4 | 0.00001 | 15,11,5 | 0.00001 |
| 2,9,1 | 0.00001 | 9,13,4 | 0.00001 | 15,13,5 | 0.00001 |
| 2,10,1 | 0.00001 | 9,18,4 | 0.00001 | 15,17,5 | 0.00001 |
| 2,12,1 | 0.729185682 | 9,19,4 | 6.701219752 | 16,9,3 | 1.171529893 |
| 2,13,1 | 0.00001 | 10,1,1 | 0.78844844 | 16,13,3 | 0.00001 |
| 2,14,1 | 0.057978612 | 10,4,1 | 0.00001 | 16,17,3 | 0.00001 |
| 2,15,1 | 0.00001 | 10,5,1 | 0.00001 | 16,2,5 | 0.00001 |
| 2,16,1 | 0.00001 | 10,6,1 | 0.495447933 | 16,3,5 | 0.00001 |
| 2,17,1 | 0.000245333 | 10,7,1 | 0.077316664 | 16,4,5 | 0.542604309 |
| 3,2,1 | 0.00001 | 10,12,1 | 0.00001 | 16,5,5 | 0.610990935 |
| 3,4,1 | 0.00001 | 10,13,1 | 0.00001 | 16,6,5 | 0.00001 |
| 3,5,1 | 0.623040257 | 10,15,1 | 0.00001 | 16,7,5 | 0.00001 |
| 3,7,1 | 0.473815713 | 10,16,1 | 0.04647388 | 16,10,5 | 0.00001 |
| 3,9,1 | 0.00001 | 10,17,1 | 0.00001 | 16,11,5 | 0.00001 |
| 3,10,1 | 0.00001 | 10,18,1 | 0.00001 | 16,12,5 | 0.00001 |
| 3,13,1 | 0.00001 | 10,9,2 | 0.409898334 | 16,13,5 | 0.00001 |
| 3,15,1 | 0.00001 | 10,19,2 | 0.590101666 | 16,14,5 | 0.00001 |
| 3,16,1 | 0.00001 | 10,2,3 | 0.00001 | 16,15,5 | 0.00001 |
| 3,17,1 | 0.00001 | 11,9,1 | 0.00001 | 16,17,5 | 2.557529682 |
| 3,19,1 | 0.00001 | 11,15,1 | 0.031952411 | 16,18,5 | 0.00001 |
| 4,5,2 | 0.00001 | 11,16,1 | 0.00001 | 17,18,1 | 1.98119043 |
| 4,8,2 | 0.00001 | 11,17,1 | 0.280944085 | 17,12,3 | 13.09743929 |
| 4,9,2 | 0.00001 | 12,1,1 | 0.00001 | 17,1,6 | 0.00001 |
| 4,10,2 | 0.00001 | 12,4,1 | 0.00001 | 17,2,6 | 0.00001 |
| 4,13,2 | 2.647081833 | 12,6,1 | 0.299650223 | 17,3,6 | 0.00001 |
| 4,15,2 | 0.00001 | 12,7,1 | 0.00001 | 17,4,6 | 1.622582674 |
| 4,16,2 | 0.00001 | 12,9,1 | 0.00001 | 17,7,6 | 0.00001 |

(continued)

| l,m,k | $a_{lm}^{k}$ | l,m,k | $a_{lm}^{k}$ | l,m,k | $a_{lm}^{k}$ |
|---|---|---|---|---|---|
| 4,5,3 | 0.206865342 | 12,10,1 | 0.598572658 | 17,9,6 | 0.00001 |
| 4,9,3 | 0.872406815 | 12,13,1 | 0.00001 | 17,10,6 | 0.12939025 |
| 5,4,2 | 2.059488775 | 12,6,2 | 6.684004703 | 17,11,6 | 0.00001 |
| 5,8,2 | 0.00001 | 13,2,1 | 0.00001 | 17,12,6 | 0.00001 |
| 5,13,2 | 0.00001 | 13,3,1 | 0.00001 | 17,13,6 | 0.00001 |
| 5,15,2 | 0.00001 | 13,4,1 | 0.00001 | 17,15,6 | 0.358366182 |
| 5,16,2 | 0.00001 | 13,5,1 | 0.00001 | 17,16,6 | 0.00001 |
| 5,4,3 | 4.126162673 | 13,7,1 | 0.00001 | 17,18,6 | 0.00001 |
| 6,13,2 | 0.08925795 | 13,9,1 | 0.00001 | 18,1,4 | 0.811337981 |
| 6,2,3 | 0.00001 | 13,10,1 | 1.067921056 | 18,2,4 | 0.266331011 |
| 6,9,3 | 0.00001 | 13,12,1 | 0.00001 | 18,3,4 | 0.00001 |
| 6,10,3 | 2.150263121 | 13,14,1 | 0.00001 | 18,5,4 | 0.31865168 |
| 6,11,3 | 2.638237891 | 13,19,1 | 0.00001 | 18,6,4 | 0.301211457 |
| 6,12,3 | 0.00001 | 14,4,1 | 20.00391639 | 18,9,4 | 0.00001 |
| 6,13,3 | 0.00001 | 14,5,1 | 0.00001 | 18,10,4 | 0.00001 |
| 7,9,2 | 0.00001 | 14,8,1 | 4.072279433 | 18,12,4 | 0.00001 |
| 7,10,2 | 0.444582668 | 14,15,1 | 0.00001 | 18,13,4 | 0.414572907 |
| 8,4,2 | 1.421938284 | 15,9,2 | 0.00001 | 19,3,1 | 0.00001 |
| 8,5,2 | 0.00001 | 15,11,2 | 11.00280603 | 19,10,1 | 1.006053498 |
| 8,11,2 | 0.00001 | 15,13,2 | 0.308034705 | 19,3,4 | 0.080680453 |
| 8,13,2 | 0.00001 | 15,16,2 | 1.017407913 | 19,7,4 | 0.173416264 |
| 8,14,2 | 0.451198906 | 15,9,3 | 0.881333411 | 19,10,4 | 0.041835704 |
| 8,15,2 | 0.00001 | 15,13,3 | 0.059769381 | 19,13,3 | 0.013215266 |
| 9,6,1 | 1.095052834 | 15,17,3 | 0.058897208 | | |
| 9,13,1 | 0.00001 | 15,2,5 | 0.00001 | | |

**Table 7.** Estimated non-dimensionalized system parameters $a_{lm}^{k}$ (l,m,k = Origin, Target, Neurotransmitter).

| l,m,k | $a_{lm}^{k}$ | l,m,k | $a_{lm}^{k}$ | l,m,k | $a_{lm}^{k}$ |
|---|---|---|---|---|---|
| 1,3,1 | -2.582714128 | 9,19,2 | 1.296085287 | 15,4,5 | 1.473369783 |
| 1,9,1 | 3.098354921 | 9,2,3 | -2.393370253 | 15,5,5 | 1.877446866 |
| 1,13,1 | 0.484359207 | 9,7,4 | -8.577215783 | 15,6,5 | -0.541324404 |
| 2,3,1 | -2.068867096 | 9,10,4 | -7.837159899 | 15,7,5 | -0.592545443 |
| 2,4,1 | 0.2081835 | 9,11,4 | 1.496674341 | 15,10,5 | -0.490103365 |
| 2,5,1 | 0.201145347 | 9,12,4 | -1.223850552 | 15,11,5 | -0.854341863 |
| 2,9,1 | -0.328072993 | 9,13,4 | -7.60195598 | 15,13,5 | -0.916945354 |
| 2,10,1 | 2.518982159 | 9,18,4 | -4.775748273 | 15,17,5 | 2.241685363 |
| 2,12,1 | -1.158881313 | 9,19,4 | 29.51925615 | 16,9,3 | 0.30697923 |

(continued)

| l,m,k | a_lm^k | l,m,k | a_lm^k | l,m,k | a_lm^k |
|-------|--------|-------|--------|-------|--------|
| 2,13,1 | -1.129053985 | 10,1,1 | -0.574112718 | 16,13,3 | 1.124172553 |
| 2,14,1 | 1.395982793 | 10,4,1 | -0.00586772 | 16,17,3 | -0.431151782 |
| 2,15,1 | 0.463865193 | 10,5,1 | -0.032521269 | 16,2,5 | -1.084815175 |
| 2,16,1 | 1.565928205 | 10,6,1 | 1.025492596 | 16,3,5 | 0.892741532 |
| 2,17,1 | -0.669211809 | 10,7,1 | -0.487395476 | 16,4,5 | 3.91037434 |
| 3,2,1 | -10.39894323 | 10,12,1 | -5.18295445 | 16,5,5 | 4.812813159 |
| 3,4,1 | 1.550222941 | 10,13,1 | -5.069998093 | 16,6,5 | -0.589109345 |
| 3,5,1 | 1.543076036 | 10,15,1 | 0.962401151 | 16,7,5 | -0.703502998 |
| 3,7,1 | 1.421105682 | 10,16,1 | -3.609223888 | 16,10,5 | -0.474715692 |
| 3,9,1 | 1.005680325 | 10,17,1 | 1.619808411 | 16,11,5 | -1.288181669 |
| 3,10,1 | -2.516757977 | 10,18,1 | 8.745147569 | 16,12,5 | -1.792792532 |
| 3,13,1 | 0.192322742 | 10,9,2 | -2.43829374 | 16,13,5 | -1.427996134 |
| 3,15,1 | 1.809855375 | 10,19,2 | 3.43829374 | 16,14,5 | -2.021673276 |
| 3,16,1 | 2.928947235 | 10,2,3 | 4.156237573 | 16,15,5 | -2.341592402 |
| 3,17,1 | 0.659270303 | 11,9,1 | -4.400843677 | 16,17,5 | 5.626279136 |
| 3,19,1 | 2.805220571 | 11,15,1 | -1.512488151 | 16,18,5 | -1.548653071 |
| 4,5,2 | -5.728059911 | 11,16,1 | 2.506954081 | 17,18,1 | 0.172546989 |
| 4,8,2 | -7.953882153 | 11,17,1 | -0.879351397 | 17,12,3 | 2.112501646 |
| 4,9,2 | 13.65644597 | 12,1,1 | 0.536071892 | 17,1,6 | -0.275088845 |
| 4,10,2 | 4.46174265 | 12,4,1 | 0.868195923 | 17,2,6 | 0.483362538 |
| 4,13,2 | 4.99224376 | 12,6,1 | 1.470998495 | 17,3,6 | -0.152757976 |
| 4,15,2 | -8.410460862 | 12,7,1 | 0.586755805 | 17,4,6 | 2.159295433 |
| 4,16,2 | -0.018029451 | 12,9,1 | -0.31875723 | 17,7,6 | -0.128291803 |
| 4,5,3 | 0.207747848 | 12,10,1 | -7.996704059 | 17,9,6 | -0.152757976 |
| 4,9,3 | -0.207747848 | 12,13,1 | 5.982930746 | 17,10,6 | 0.63015958 |
| 5,4,2 | -9.235064088 | 12,6,2 | 7.91866215 | 17,11,6 | -0.103825629 |
| 5,8,2 | 1.580614541 | 13,2,1 | -7.021659502 | 17,12,6 | -0.103825629 |
| 5,13,2 | 4.060521381 | 13,3,1 | -0.263681283 | 17,13,6 | -0.164991063 |
| 5,15,2 | 1.493154194 | 13,4,1 | 1.357792854 | 17,15,6 | 0.366992605 |
| 5,16,2 | 3.100773972 | 13,5,1 | 1.352781027 | 17,16,6 | -0.498324397 |
| 5,4,3 | 4.124577446 | 13,7,1 | 1.2672483 | 17,18,6 | -0.299555018 |
| 6,13,2 | -1.145399497 | 13,9,1 | 0.975927807 | 18,1,4 | 1.428361404 |
| 6,2,3 | -5.041673277 | 13,10,1 | -1.494211406 | 18,2,4 | 0.652078032 |
| 6,9,3 | 3.337980447 | 13,12,1 | 0.384314162 | 18,3,4 | -2.268961908 |
| 6,10,3 | 2.176240389 | 13,14,1 | 2.203617641 | 18,5,4 | 0.726601236 |
| 6,11,3 | 2.656771058 | 13,19,1 | 2.2378704 | 18,6,4 | 0.701760168 |
| 6,12,3 | 2.91235466 | 14,4,1 | -0.229957455 | 18,9,4 | -4.495480692 |
| 6,13,3 | -5.041673277 | 14,5,1 | -4.244778615 | 18,10,4 | 1.306713509 |

(continued)

| l,m,k | $a_{lm}{}^k$ | l,m,k | $a_{lm}{}^k$ | l,m,k | $a_{lm}{}^k$ |
|---|---|---|---|---|---|
| 7,9,2 | 0.268071556 | 14,8,1 | 9.463412854 | 18,12,4 | 0.508531849 |
| 7,10,2 | -4.518058786 | 14,15,1 | -4.21863424 | 18,13,4 | 2.4403964 |
| 8,4,2 | -11.82143057 | 15,9,2 | 1.10657325 | 19,3,1 | 0.554835807 |
| 8,5,2 | 2.160661805 | 15,11,2 | 23.79745856 | 19,10,1 | 0.445164193 |
| 8,11,2 | 6.778907202 | 15,13,2 | 0.441696806 | 19,3,4 | 0.473092682 |
| 8,13,2 | 6.778907202 | 15,16,2 | -0.548270056 | 19,7,4 | 0.526907318 |
| 8,14,2 | 1.055196711 | 15,9,3 | -1.46641197 | 19,10,4 | 0.110643995 |
| 8,15,2 | 1.521595433 | 15,13,3 | 1.803032777 | 19,13,3 | -0.911756467 |
| 9,6,1 | 1.302011008 | 15,17,3 | 0.663379193 | | |
| 9,13,1 | -0.302011008 | 15,2,5 | -0.763282238 | | |

3. Neuroactive compound Functions

Simulations of the mathematical model with the above-mentioned parameters resemble the dynamical behavior of the extracellular local concentrations of the neurotransmitter under baseline conditions. In order to simulate the effects of neuropsychiatric neuroactive compounds on the extracellular local concentrations of the neurotransmitter, the main equation of the model is modified. Let $K_{p.u}$ denote the ratio of unbound (active) brain neuroactive compound concentration to its plasma concentration, which is either obtained *in vivo* or by forward dosimetry and physiologically-based pharmacokinetics pharmacologically-based pharmacokinetics (PBPK) modeling. Then the function $c(t)=K_{p,u} P(t)$, with $P(t)$ denoting the time-course of blood neuroactive compound concentration, represents the time-course of the concentration of unbound (active) neuroactive compound in the rodent brain. Based on the assumption that the effects of the neuroactive compound on the neurochemical systems correlate with its active concentration in the brain, the impact of the neuroactive compound will be modeled as $\delta_{i,r}^{k} c_{r}^{k}(t)$.

Since the procedure is designed to simulate not only singular neuroactive compound events but also combinatorial administration of different substances at the same time, the index $r$ (and $w$) is proposed to distinguish the different substances that are co-administered and act on the same neurotransmitter system $k$. $\delta_{i,r}^{k}$ as measures for the "potency" of the neuroactive compounds are non-zero real valued parameters, which translate the active concentration of the neuroactive compounds into an effective dimension with respect to the local concentration $s_{i}^{k}(t)$ of the neurotransmitter $k$ in brain region $j$. Depending on the different mechanisms of action of neuroactive compounds e.g. agonism, antagonism or direct action on the extracellular local concentrations of the neurotransmitter (reuptake or synthesis manipulators) the terms $\delta_{i,r}^{k} c_{r}^{k}(t)$ are embedded into the mathematical model either within the structure of the neurochemical balance functions or as an additional term within the main equations and the parameters $\delta_{i,r}^{k}$ are calibrated by nonlinear greybox parameter estimations with reference to experimentally obtained microdialysis experiments measuring the neuroactive compound-induced alterations of the neurotransmitter concentrations in either 1) one brain regions and three different doses of the neuroactive compound; 2) two brain regions and two different doses of the neuroactive compound or 3) two brain regions with acute and chronic administration of a single dose of the neuroactive compound of interest.

To study the combinatorial effects of different neuroactive compounds simultaneously, the parameters $\delta_{i,r}^{k}$ have to be estimated in advance for each substance and then the different terms are integrated into the model components. In the following, the exact integration of the neuroactive compound-specific terms into the model based on the mechanisms of action of neuroactive compounds is elaborated thoroughly.

3.1. Agonists and Antagonists

As discusses in the context of the mathematical model for the release, the interaction of different neurochemical systems and the activation of a brain region as the consequence of its neurochemical afferents are modeled by the saturation function $\Gamma_l(t) \in [0, 1]$ with

$$\Gamma_l(t) = 1 - \frac{1}{e^{4F(t)} + 1}$$

,

the so-called activity function, which in a similar manner as the hysteresis-like operators acts as a switch for neurotransmitter dynamics. These components of the mathematical model depend on the dynamical behavior of $F_l(t)=A_l(t)-B_l(t)$, the neurochemical balance function at node $l$. Since the terms $A_l(t)$ and $B_l(t)$ represent the impact of excitatory and inhibitory afferents to the region $l$ respectively, their modifications with the neuroactive compound terms $\pm \sum_{r=1}^{n} \delta_{j,r}^{k} \, c_r^k(t)$ and $\pm \sum_{w=1}^{m} \delta_{n,w}^{q} \, c_w^q(t)$ imply the action of agonists and antagonists on the neurotransmitter systems $k$ and q respectively, in a pharmacologically plausible manner.

$$A_l(t) := \frac{1}{Ex(l)} \sum_{j=1}^{Ex(l)} \omega^k \frac{s_j^k(t) - s_j^k(0) \pm \sum_{r=1}^{n} \delta_{j,r}^{k} \, c_r^k(t)}{s_j^k(0)}$$

$$B_l(t) := \frac{1}{Inh(l)} \sum_{p=1}^{Inh(l)} \omega^q \frac{s_p^q(t) - s_p^q(0) \pm \sum_{w=1}^{m} \delta_{p,w}^{q} \, c_w^q(t)}{s_p^q(0)}$$

,

represent the modified excitatory and inhibitory components of the activation process. Since the activity function $\Gamma_l(t)$ resembles a biological switch as a function of the neurochemical balance of afferents at region $l$, the sign of the terms $\pm \sum_{r=1}^{n} \delta_{j,r}^{k} \, c_r^k(t)$ and $\pm \sum_{w=1}^{m} \delta_{n,w}^{q} \, c_w^q(t)$ denote the agonism (+) and antagonism (-) of the action of the respective neurotransmitter $s_i^k(t)$. Through the positive/negative sign of the neuroactive compound component the values of $A_l(t)$ will increase/decrease, which in turn induces "turns on/off " the switch and activates/deactivates the region $l$. Analogously, through the positive/negative sign of the neuroactive compound component the values of $B_l(t)$ will increase/decrease, which thereby induces "turns off/on " the switch and deactivates/activates the region $l$.

3.2. Direct Manipulations of Neurotransmitter Concentrations [Re-uptake or Synthesis Processes]

Neuroactive compounds that directly manipulate the extracellular local concentrations of the neurotransmitter such as the selective serotonin re-uptake inhibitors (SSRIs) are integrated into the vector fields of the main equations of the model by the terms $\sigma^k(t) = \pm \sum_{h=1}^{N} \delta_{l,h}^{k} \, c_h^k(t)$:

$$\frac{ds_l^k(t)}{dt} = \sum_{\substack{m=1 \\ m \neq l}}^{deg^{out}(l)} -f_{lm}^k(t)s_l^k(t) + r_l^k(t-\tau_{lm})s_m^k(t-\tau_{lm}) + \sum_{\substack{j=1 \\ j \neq l}}^{deg^{in}(l)} f_{jl}^k(t-\tau_{jl})s_j^k(t-\tau_{jl}) - [s_l^k(t-\tau_\alpha) - s_l^k(t-2\tau_\alpha)] + \sigma^k(t)$$

As mentioned above, the parameters $\delta_{l,h}^{k}$ are calibrated by nonlinear grey-box parameter estimations with reference to experimentally obtained microdialysis experiments measuring the neuroactive compound-induced alterations of the neurotransmitter concentrations.

### 3.3. Neuroactive compound Action under Anesthesia

It is commonly known that anesthesia have an impact on the basal extracellular local concentration of a neurotransmitter. These anesthesia-induced alterations in the basal neurotransmitter levels are critical to the invented procedure. Firstly, changes in the initial functions of the system may have significant influences on the dynamical behavior of neurochemical trajectories and secondly, anesthesia may interact with the administered neuroactive compounds such as benzodiazepines and thereby disturb the simulations of the neuroactive compound-induced effects.

Within the framework of this invention, two strategies are present to robustly overcome these issues:

1. The investigation of neuroactive compound effects under anesthesia is treated as a combinatorial pharmacological manipulation. Hereby, initial parameter estimations for the parameters $\delta_{i,r}^{k}$ of the anesthetic neuroactive compound and the neuroactive compound of interest is required in order to perform the *in silico* analysis as described above.

2. The average baseline neurotransmitter concentrations (Table 1) are replaced with the experimentally obtained anesthesia-induced basal levels and the simulations are performed using the modified values as the initial state of the model.

### 4. Numerical Simulation

The numerical simulation of the system provides 4-dimensional trajectories (local neurotransmitter concentration x time), which suggest the time course of the neurotransmitter concentrations under basal and neuroactive compound-induced conditions. Depending on the simulation setup the results are commonly expressed as matrices with 88 rows and step-size x time columns. Each row of the matrix represents the discrete time-course of the behavior of extracellular local concentration of a specific neurotransmitter in a specific brain region. By dividing the values of a row through the average of the initial functions (baseline concentration), one obtains the percentage of the concentration with respect to the basal level, which in turn reflects the neuroactive compound effects as measured and represented by *in vivo* microdialysis experiments. Since the mathematical model and its initial function do not generate derivative discontinuities, numerical simulations of the model can be performed by any of the currently available algorithms utilizing higher order Runge-Kutta-method with Hermite or $C^1$ interpolation. In particular, if the model is realized in MATLAB then the dde23 solver is the software of choice for robust simulations.

### 5. Rescaling

Rescaling refers to the simulation of the mathematical model based on baseline concentrations of individual animals, which in turn provides quantitative measures of neuroactive compound effects at systems (neurocircuitry) level. By considering the individual variabilities the outcome of the rescaling process are directly comparable with *in vivo* microdialysis measurements. Since the initial functions of the model variables are based on meta-analyses (average values of baseline concentrations), the individual variabilities are not simulated by default within the procedure. Instead, numerical simulations of the model provide 4-dimensional trajectories that reveal the dynamical behavior of the neurotransmitter concentrations in presence of neuroactive compounds, which serve as qualitative measures of the systematic effects of neuroactive compounds. In particular, knowledge of the qualitative behavior of neurotransmitter concentrations in response to neuropsychiatric neuroactive compounds provides an appropriate and reliable framework for screening of neuroactive compounds based on their systemic efficacy.

However, for quantitative analysis of neuroactive compound effects, especially for replacing *in vivo* microdialysis experiments by the invented procedure, baseline data from individual animals is required. While dual-probe *in vivo* microdialysis provides quantitative measures of neuroactive compound effects, this technology is restricted to observations in only two brain regions and a global systematic picture is not provided. Therefore, even the individual baseline values are experimentally available only for two brain regions.

Hence the rescaling process requires a combination of experimental and mathematical techniques as described below:

1. The numerical simulation is performed using the averaged initial functions, which provides the qualitative behavior of neurochemical trajectories;

2. Either by *in vivo* microdialysis, non-invasive measurements of blood oxygen and/or glucose response patterns by functional or pharmacological MRT or based on a database containing experimental values for individual animals, basal and/or drug-induced concentrations for single animals are obtained for two brain regions;

3. By using the experimental values as references for the qualitative measures in the two regions, non-linear greybox parameter estimation will be performed to rescale the remaining baseline concentrations into "individual" levels so that the simulation of the model based on the estimated basal values reveals the predicted qualitative behavior;

4. Based on the set of experimentally obtained and estimated baseline concentrations as initial states of the system, numerical simulations will be performed to predict the quantitative behavior of the neurochemical trajectories for individual animals.

6. Experimental Validation and Detremination Power

In order to validate the mathematical model and test its predictive power, numerical simulations of a variety of neuropsychoactive substances (Haloperidol i.p. 0.5 mg/kg (dopamine antagonist), Diazepam i.p. 5 mg/kg (GABA agonist), acute and chronic ethanol i.p. 1 g/kg (GABA, ACh and 5-HT agonist and glutamate antagonist), fluoxetine i.p. 10 mg/kg (selective serotonin re-uptake inhibitor)) were statistically compared with *in vivo* microdialysis experiments. In the choice of neuroactive compound samples the comprehensive representation of different functional neuroactive compound types (agonists, antagonists, agonism and antagonism of different neurotransmitter systems and re-uptake inhibition) was the main criterion. The choice of neuroactive compound dosage was on the one hand based on its non-lethality and on the other hand doses were considered that are known to induce significant alterations in extracellular local concentrations of the neurotransmitter.

6.1. Haloperidol [i.p. 0.5 mg/kg]

Haloperidol is a butyrophenone first-generation or typical antipsychotic neuroactive compound that exhibits antagonistic activity at brain dopaminergic D2 receptors with slow dissociation kinetics (Ki = 1.55 nM at D2 receptors). Therefore, haloperidol was modeled as a pure dopamine antagonist with a homogeneous neuronal distribution in the procedure (Noori and Jäger, 2009). Numerical simulations of the haloperidol-induced neurochemical dynamics (in ms-scale) shows phasic reduction of dopaminergic activity at the expected target region (corpus striatum: nucleus accumbens and caudate putamen) and further suggests that optimal (long-term) effects are most likely achieved by a combination of dopamine antagonism and reduction of dopamine synthesis.

Moreover, quantitative comparison of the simulated trajectories (in minutes-scale) with *in vivo* microdialysis measurements of haloperidol-induced effects (peak % baseline and peak time) showed no significant differences. The accuracy of the model was not only based on the monoaminergic systems but the simulations suggest enhancements in cholinergic system (almost 150% in acetylcholine concentrations in prefrontal cortex), an effect that has been experimentally observed in previous studies (Ichikawa et al. 2002).

6.2. Diazepam [i.p. 5 mg/kg]

Diazepam is a benzodiazepine that by potentiation of the activity of the $GABA_A$ receptor induces sedative, hypnotic, anxiolytic, anticonvulsant, and muscle relaxant properties. With a binding affinity (Ki) of 7 nM, diazepam acts as a very potent agonist agent on the GABAergic system. In our procedure, diazepam was modeled as a pure GABA agonist, which is homogeneously distributed within the brain regions in the neurocircuitry for modeling neuroactive compound effects. Interestingly, numerical simulations of diazepam-induced effects in the brain showed decreases in hippocampal acetylcholine (to 70% of baseline) and dopamine concentrations prefrontal cortex (to 70% of baseline), which are also observed by *in vivo* microdialysis studies. It is noteworthy that these observations reflect the ability of the model to simulate the interactions of different neurotransmitter systems in a realistic manner. Thus, programmed manipulations in a specific neurotransmitter system not only simulate the changes in the respective chemical system but also reflect all corresponding regulatory mechanisms in the rat brain.

6.3. Acute ethanol acute [i.p. 1 g/kg]

Alcohol (ethanol, $CH_3$-$CH_2$-OH), at concentrations in the 10-20 mM range, directly interferes with the function of several ion channels and receptors. In particular, the function of the glutamate receptor NMDA is inhibited by ethanol in a concentration-dependent manner over the range of 5-50 mM, a range that also produces intoxication. The amplitude of the NMDA-activated current was reduced 61 % by 50 mM ethanol. Besides the NMDA receptor, other receptors or ion channels expressed within the CNS also have putative alcohol-binding sites. In particular, the function of $GABA_A$ receptors is enhanced by ethanol. The $GABA_A$ receptor/chloride channel complex is a pentameric ligand-gated ion channel and the major inhibitory neurotransmitter receptor in the mammalian brain. Furthermore, ethanol potentiates neuronal nicotinic acetylcholine and serotonin 5-HT$_3$-receptor function.

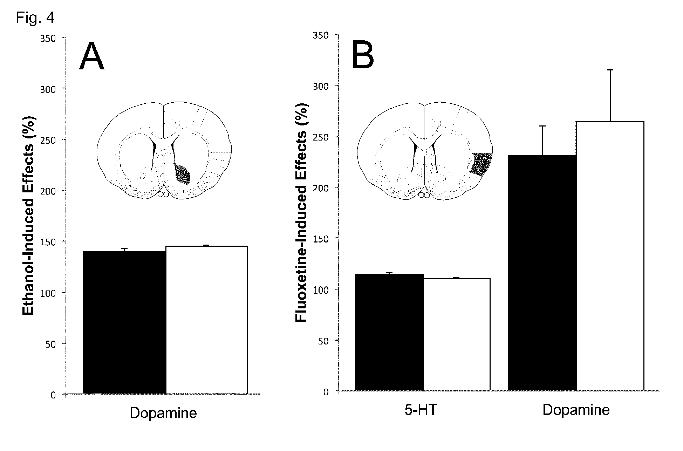

Therefore, alcohol was modeled as an agonist for GABA, acetylcholine and serotonin and simultaneously as an antagonist of the glutamatergic system with equal distribution in all brain regions. Furthermore, due to the pharmacokinetics of ethanol, it was assumed that the blood alcohol concentration is equal with the active and available brain concentration. This assumption reduces the computational effort in neuroactive compound modeling significantly. The simulations reproduced faithfully the previously measured alterations in neurotransmitter concentrations in the brain. In particular, the results suggest a global enhancement in the monoaminergic systems, an effect that has been confirmed a posteriori by a meta-analysis performed by Noori and colleagues (Brand et al. 2013). Due to the translational aspects with respect to the reward system, the response of the dopaminergic system towards acute ethanol exposure was of special interest. The simulations show an increase of 140-150% in the dopamine concentrations of nucleus accumbens, which is in complete agreement with *in vivo* microdialysis measurements under equal conditions (Figure 4A).

6.4. <u>Chronic ethanol (post-dependent model of alcoholism)</u>

Due to neuroadaptive mechanisms and systemic rearrangements in information processing pathways, the acute effects of neuroactive compounds do not necessarily converge into the chronic systems responses. Therefore, it is crucial to investigate the capabilities of the procedure in characterizing and predicting the effects of chronic neuroactive compound exposure on the neurochemical systems. Since invasive techniques such as *in vivo* microdialysis do not allow a continuous observation of the neurotransmitter activity for a time period longer than 72 hours, the success of this procedure will provide a new possibility to capture the dynamics of neuroactive compound-induced adaptations. This information is crucial for neuroactive compound discovery and development processes because these substance-induced adaptations are directly associated with tolerance towards medication and even addiction.

For this purpose, the effect of repetitive cyclic exposure to ethanol (post-dependent state) was simulated with the procedure and the predictions were experimentally validated. Since no information on the post-dependent neurochemical responses was available in advance, the outcome of the procedure may be considered as "blind" predictions. Transition to ethanol dependence involves long-term neuroadaptations that lead to excessive voluntary ethanol intake and altered responses to stress.

Long-lasting neural and behavioral plasticity thought to model this process has been observed in laboratory rats following a history of dependence induced by prolonged exposure to ethanol vapor. Repeated cycles of intoxication and withdrawal, which mimic the course of the clinical condition is the most effective paradigm for inducing these long-term neuroadaptations, together labeled as "the post-dependent state". The post-dependent state is induced as described in the following: Exposure paradigm was: 1 week of habituation to the chambers (no alcohol), 1 week of continuous exposure to 22 mg/L alcohol to adapt to the novel odor, 7 weeks of exposure to alcohol vapor adjusted to produce blood alcohol concentrations of 150-320 mg/dl for 17 hours (4 pm-9 am) each day. Ethanol was modeled as described above as an agonist for GABA, acetylcholine and serotonin and simultaneously as an antagonist of the glutamatergic system with a periodic non-linear active concentration pattern, which reflects blood alcohol concentrations based on the post-dependent exposure paradigm.

The simulations suggest neuroadaptations in the glutamatergic and GABAergic systems such as switches in basal concentrations of glutamate in amygdala (+150% of baseline), prefrontal cortex (+200% of baseline), and hippocampus (-200% of baseline) as well as in basal GABA level in nucleus accumbens (+200% of baseline). In contrast to the common belief, the simulations further suggest an enhancement in the basal dopamine levels in nucleus accumbens of around 150%. Although surprising, this effect was a posteriori confirmed by Sommer and colleagues (Figure 3, unpublished data) experimentally.

6.5. <u>Fluoxetine 10 mg/kg</u>

The selective serotonin reuptake inhibitors (SSRIs) with high affinity to serotonin uptake sites, low affinity to noradrenaline uptake sites, and very low affinity for neurotransmitter receptors were the result of these efforts. SSRIs are thus the first class of rationally designed therapeutic neuroactive compounds in psychiatry. Moreover, the use of SSRIs was extended from major depression to minor depression and other psychiatric disorders that are also suggested to be associated with a dysfunctional state of the serotonin system. This includes anxiety, obsessive-compulsive disorders, or premenstrual dysphoric disorders. Thus, the use of SSRIs is a rational, mechanism-based therapy. In most countries, fluoxetine was the first SSRI that became available for clinical use. It is a racemic mixture of two enantiomers, whereby the S-enantiomer is almost 1.5 times more potent in the inhibition of serotonin reuptake than the R-enantiomer. Fluoxetine exhibits nonlinear kinetics, indicated by a disproportionate increase in its blood concentrations after dose escalation. Therefore, fluoxetine was modeled as an agent acting directly on the serotonin concentrations with a homogeneous distribution. For this particular investigation, the monoamine responses in insular cortex were a posteriori measured in insular cortex for the first time (blind prediction test). Both methods *(in silico* and *in vivo)* showed no significant changes in the serotonin levels, while the dopamine concentration was increased around 270% (Figure 4B).

6.6. Summary

In summary, these tests demonstrate the appropriateness and efficacy of the invented procedure to predict acute and chronic neuroactive compound effects on the extracellular local concentrations of the neurotransmitter in rodents. Most importantly, the procedure results do not differ significantly from the *in vivo* microdialysis measurements. Therefore, the procedure provides a fast and low-cost alternative to the expensive in vivo technologies utilized in preclinical testing phase of novel pharmaceutical substances.

Further references of interest are the following articles:

Noori H.R., Spanagel R., Hansson A., Neurocircuitry for Modeling Neuroactive compound Effects, 2012. Addict Biol 17(5):827-864.

Noori H.R., The Effects of the Acute Administration of Low-Dosage Ethanol on the Phasic Neurochemical Oscillations of the Basal Ganglia, 2011. Math Med Biol 29(3):231-244.

Noori H.R. and Jäger W., Neurochemical Oscillations in the Basal Ganglia, 2010. Bull Math Biol 72(1):133-147.

**Claims**

**1.** A computer-implemented method for providing a set of clinical data on extracellular local concentrations of one or more neurotransmitters in the absence or presence of one or more neuroactive compounds in a set of neuronal regions, said method comprising the steps of:

(i) providing clinical data on evolution of extracellular local concentrations of the one or more neurotransmitters in the absence and/or presence of neuroactive compounds in a reduced number of neuronal regions from the set of neuronal regions;
(ii) optionally providing the initial concentrations of the one or more neuroactive compounds in each neuronal region of the set of neuronal regions;
(iii) simulating an evolution of extracellular local concentrations of one or more neurotransmitters within a neurocircuitry modelling the extracellular local concentrations of said one or more neurotransmitters in the absence or optionally in the presence of neuroactive compounds and the effect of said initial concentrations of said one or more neuroactive compounds,
wherein the basal extracellular local concentrations of said one or more neurotransmitters are averaged basal extracellular local concentrations; and
(iv) rescaling the simulated evolution of extracellular local concentrations of the one or more neurotransmitters by the clinical data on evolution of extracellular local concentrations of the one or more neurotransmitters in the absence and/or presence of neuroactive compounds in a reduced number of neuronal regions,

whereby the clinical data on evolution of extracellular local concentrations of the one or more neurotransmitters in the absence and/or presence of neuroactive compounds in the reduced number of neuronal regions is extended to clinical data on extracellular local concentrations of the one or more neurotransmitters in the absence or presence of one or more neuroactive compounds for each neuronal region of the set of neuronal regions.

**2.** The computer-implemented method according to claim 1, wherein the neurocircuitry comprises a set of coupled differential equations, the coupling reflecting at least interactions of the one or more neurotransmitters between one neuronal region and another neuronal region, preferably wherein the number of coupled differential equations is proportional to the number of neuronal regions in the set of neuronal regions and/or proportional to the number of neurotransmitters.

**3.** The computer-implemented method according to any one of the preceding claims, wherein the neurocircuitry comprises a term reflecting release, the term reflecting release, for a determined neurotransmitter in a determined neuronal region, being correlative to the extracellular local concentration of said determined neurotransmitter in said determined neuronal region.

**4.** The computer-implemented method according to claim 4, wherein the term reflecting release comprises a correlating factor being itself proportional to a saturation function.

5. The computer-implemented method according to any one of the preceding claims, wherein the neurocircuitry comprises a term reflecting resorption, said term reflecting resorption, for a determined neurotransmitter in a determined neuronal region, being correlative to the extracellular local concentration of said determined neurotransmitter in a neuronal region different from said determined region at earlier times.

6. The computer-implemented method according to any one of the preceding claims, wherein the neurocircuitry comprises a term reflecting afferent projections, the term reflecting afferent projections, for a determined neurotransmitter in a determined neuronal region, being correlative to an amount of said determined neurotransmitter in a neuronal region different from the determined region at earlier times.

7. The computer-implemented method according to any one of the preceding claims, wherein the neurocircuitry comprises a term reflecting regeneration of the one or more neurotransmitters.

8. The computer-implemented method according to any one of the preceding claims, wherein the neurocircuitry comprises a term reflecting the effect of one or more agonists and/or antagonists on the activity of the one or more neurotransmitters.

9. The computer-implemented method according to any one of the preceding claims, wherein the neurocircuitry comprises a term reflecting the effect of anesthesia.

10. The computer-implemented method according to any one of the preceding claims, wherein the neuroactive compound is a neuropsychoactive compound and/or the neuronal region is a brain region.

11. The computer-implemented method according to any one of the preceding claims, wherein at least one of the one or more neurotransmitters is selected from the group consisting of glutamate, gamma-aminobutyrate, acetylcholine, dopamine, norepinephrine and serotonin.

12. The computer-implemented method according to any one of the preceding claims, wherein the set of neuronal regions is present in a subject suffering from or being at risk of a neuropathologic condition.

13. The computer-implemented method according to any one of the preceding claims, wherein said method is a screening method wherein at least one local and/or systemic effect of a compound or a combination of compounds selected from a compound library is determined.

14. A computer-implemented method for providing a set of clinical data on extracellular local concentrations of one or more neurotransmitters in the absence or presence of one or more neuroactive compounds in a set of neuronal regions, said method comprising the steps of:

(i) providing clinical data on evolution of extracellular local concentrations of the one or more neurotransmitters in the absence and/or presence of neuroactive compounds in a reduced number of neuronal regions from the set of neuronal regions;
(ii) optionally providing the initial concentrations of the one or more neuroactive compounds in each neuronal region of the set of neuronal regions;
(iii) simulating an evolution of extracellular local concentrations of one or more neurotransmitters within a neurocircuitry modelling the extracellular local concentrations of said one or more neurotransmitters in the absence or optionally in the presence of neuroactive compounds and the effect of said initial concentrations of said one or more neuroactive compounds,
wherein the basal extracellular local concentrations of said one or more neurotransmitters are averaged basal extracellular local concentrations; and
(iv) rescaling the simulated evolution of extracellular local concentrations of the one or more neurotransmitters by the clinical data on evolution of blood oxygen and/or glucose levels in the absence and/or presence of neuroactive compounds in a reduced number of neuronal regions,

whereby the clinical data on evolution of blood oxygen and/or glucose levels in the absence and/or presence of neuroactive compounds in the reduced number of neuronal regions is extended to clinical data on extracellular local concentrations of the one or more neurotransmitters in the absence or presence of one or more neuroactive compounds for each neuronal region of the set of neuronal regions,
preferably wherein the method is further **characterized by** any of claims 2 to 13.

**15.** A composition comprising one or more neuroactive compounds for use in a method for treating or preventing a subject suffering from or being at risk of a neuropathologic condition, wherein said subject is administered with an amount of said composition sufficient to restore the neurocircuitry associated with said neuropathologic condition to a non-pathologic state, said amount determined by:

(i) providing clinical data on evolution of extracellular local concentrations of one or more neurotransmitters associated with said neuropathologic condition in the absence and/or presence of neuroactive compounds in a reduced number of neuronal regions from the set of neuronal regions associated with said neuropathologic condition in said subject according to the method according to any of claims 1 to 14;
(ii) simulating an evolution of extracellular local concentrations of the one or more neurotransmitters within a neurocircuitry modelling the extracellular local concentrations of said one or more neurotransmitters in the presence of various amounts of said composition and the effect of the resulting initial concentrations of said one or more neuroactive compounds according to the method according to any of claims 1 to 14; and
(iii) selecting the amount of said composition sufficient to restore the neurocircuitries to a non-pathologic state.

**16.** A computer program product directly loadable into the internal memory of a digital computer, comprising software code portions suitable for implementing the provision of a set of clinical data on extracellular local concentrations of one or more neurotransmitters in the absence or presence of one or more neuroactive compounds in a set of neuronal regions according to any one of the claims 1 to 14 when said product is run on a computer.

Fig. 1

Fig. 2

| | OS | PFC | Ins | Acb | CPu | S | BST | GP | HyT | Amy | Hb | Hc | Th | STh | SN | VTA | R | LC | Pons |

Mood disorders

Anxiety disorders

Addiction

Drug effects

Fig. 3

Fig. 4

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | NOORI H. R. ET AL: "Neurocircuitry for modeling drug effects", ADDICTION BIOLOGY, vol. 17, no. 5, 1 September 2012 (2012-09-01), pages 827-864, XP055127784, ISSN: 1355-6215, DOI: 10.1111/j.1369-1600.2012.00485.x * the whole document * | 1-16 | INV. G06F19/00 ADD. G06F19/12 |
| X | BRAND I. ET AL: "Global Ethanol-Induced Enhancements of Monoaminergic Neurotransmission: A Meta-Analysis Study", ALCOHOLISM: CLINICAL & EXPERIMENTAL RESEARCH, vol. 37, no. 12, 28 June 2013 (2013-06-28), pages 2048-2057, XP055128417, ISSN: 0145-6008, DOI: 10.1111/acer.12207 * abstract * * page 2048, left-hand column, line 1 - right-hand column, line 20 * * page 2049, right-hand column, lines 20-53 * | 1-16 | |
| A | US 2009/306944 A1 (WILLMANN STEFAN [DE] ET AL) 10 December 2009 (2009-12-10) * abstract * * figure 1 * * paragraph [0015] - paragraph [0017] * * paragraph [0020] * * paragraph [0031] - paragraph [0034] * | 15 | |
| A | US 2009/076019 A1 (TYERS MIKE [CA] ET AL) 19 March 2009 (2009-03-19) * figure 1 * * paragraph [0003] - paragraph [0029] * * paragraphs [0113], [0121] * | 15 | |

TECHNICAL FIELDS SEARCHED (IPC)

G06F

-/--

The present search report has been drawn up for all claims

| Place of search | Date of completion of the search | Examiner |
|---|---|---|
| The Hague | 17 July 2014 | Godzina, Przemyslaw |

EPO FORM 1503 03.82 (P04C01)

Europäisches
Patentamt

European
Patent Office

Office européen
des brevets

**EUROPEAN SEARCH REPORT**

Application Number

EP 14 16 1717

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GELFUSO E. A. ET AL: "Anxiety: A Systematic Review of Neurobiology, Traditional Pharmaceuticals and Novel Alternatives from Medicinal Plants", CNS & NEUROLOGICAL DISORDERS, vol. 13, no. 1, 1 February 2014 (2014-02-01), pages 150-165, XP055128289, ISSN: 1871-5273, DOI: 10.2174/18715273113129990102 * the whole document * | 15 | |
| A | RIHEL J. ET AL: "Behavioral screening for neuroactive drugs in zebrafish", DEVELOPMENTAL NEUROBIOLOGY, vol. 72, no. 3, 12 May 2011 (2011-05-12), pages 373-385, XP055129181, ISSN: 1932-8451, DOI: 10.1002/dneu.20910 * the whole document * | 13 | |

TECHNICAL FIELDS
SEARCHED (IPC)

The present search report has been drawn up for all claims

| Place of search | Date of completion of the search | Examiner |
|---|---|---|
| The Hague | 17 July 2014 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 14 16 1717

This annex lists the patent family members relating to the patent documents cited in the above-mentioned European search report.
The members are as contained in the European Patent Office EDP file on
The European Patent Office is in no way liable for these particulars which are merely given for the purpose of information.

17-07-2014

| Patent document cited in search report | Publication date | Patent family member(s) | Publication date |
|---|---|---|---|
| US 2009306944 A1 | 10-12-2009 | CA 2660618 A1<br>DE 102006028232 A1<br>EP 2035985 A1<br>US 2009306944 A1<br>WO 2007147539 A1 | 27-12-2007<br>27-12-2007<br>18-03-2009<br>10-12-2009<br>27-12-2007 |
| US 2009076019 A1 | 19-03-2009 | CA 2606658 A1<br>US 2009076019 A1 | 13-04-2008<br>19-03-2009 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **NOORI H.R. et al.** *Addict Biol,* 2012, vol. 17 (5), 827-864
- **NOORI H.R.** *Math Med Biol,* 2011, vol. 29 (3), 231-244
- **NOORI H.R. ; JÄGER W.** *Bull Math Biol,* 2010, vol. 72 (1), 133-147
- **NOORI et al.** *Addict Biol,* 2012, vol. 17 (5), 827-864

- **NOORI H.R. ; SPANAGEL R. ; HANSSON A.** Neurocircuitry for Modeling Neuroactive compound Effects. *Addict Biol,* 2012, vol. 17 (5), 827-864
- **NOORI H.R.** The Effects of the Acute Administration of Low-Dosage Ethanol on the Phasic Neurochemical Oscillations of the Basal Ganglia. *Math Med Biol,* 2011, vol. 29 (3), 231-244
- **NOORI H.R. ; JÄGER W.** Neurochemical Oscillations in the Basal Ganglia. *Bull Math Biol,* 2010, vol. 72 (1), 133-147